# EUROPEAN PATENT SPECIFICATION

(11) **EP 2 155 878 B1**
(45) Date of publication and mention of the grant of the patent: **24.02.2016**
(21) Application number: 08771306.1
(22) Date of filing: 18.06.2008
(51) Int. Cl.: A61K 38/00, A61K 48/00, C07K 14/705, C07K 14/515, C07K 14/47

(54) **A METHOD OF TREATING ABNORMAL ANGIOGENESIS VIA THE BAI FAMILY OF PROTEINS AND THEIR PROTEIN FRAGMENTS**
VERFAHREN ZUR BEHANDLUNG ABNORMER ANGIOGENESEN DURCH PROTEINE UND PROTEINFRAGMENTE DER BAI-FAMILIE
PROCÉDÉ DE TRAITEMENT D'UNE ANGIOGENÈSE ANORMALE VIA LA FAMILLE BAI DES PROTÉINES ET LEURS FRAGMENTS DE PROTÉINE

(30) Priority: 19.06.2007 US 936196 P
(43) Date of publication of application: 24.02.2010
(73) Proprietor: Emory University, Atlanta, GA 30322 (US)
(72) Inventor: VAN MEIR, Erwin, Tucker, GA 30084 (US)
(74) Representative: Vidon Brevets & Stratégie
(86) International application number: PCT/US2008/067276
(87) International publication number: WO 2008/157572

(56) References cited:
- WO-A2-00/47622
- DATABASE UniProt [Online] 22 August 2006 (2006-08-22), "SubName: Full=Bai1 protein; SubName: Full=Uncharacterized protein;", XP000002658077, retrieved from EBI accession no. UNIPROT:Q14BI4 Database accession no. Q14BI4
- DATABASE EMBL [Online] 1 July 2006 (2006-07-01), "Mus musculus brain-specific angiogenesis inhibitor 1, mRNA (cDNA clone IMAGE:40086881), complete cds.", XP000002658078, retrieved from EBI accession no. EMBL:BC115835 Database accession no. BC115835
- JEONG TAE KOH ET AL: "Extracellular fragment of brain-specific angiogenesis inhibitor 1 suppresses endothelial cell proliferation by blocking ávâ5 integrin", EXPERIMENTAL CELL RESEARCH, ACADEMIC PRESS, US, vol. 294, no. 1, 10 March 2004 (2004-03-10), pages 172-184, XP003024781, ISSN: 0014-4827, DOI: 10.1016/J.YEXCR.2003.11.008 [retrieved on 2003-12-16]
- TOLSMA S S ET AL: "PEPTIDES DERIVED FROM TWO SEPARATE DOMAINS OF THE MATRIX PROTEIN THROMBOSPONDIN-1 HAVE ANTI-ANGIOGENIC ACTIVITY", THE JOURNAL OF CELL BIOLOGY, ROCKEFELLER UNIVERSITY PRESS, US, vol. 122, no. 2, 1 July 1993 (1993-07-01), pages 497-511, XP000578336, ISSN: 0021-9525, DOI: 10.1083/JCB.122.2.497
- GRANT M A ET AL: "Structure and function of angiogenesis inhibitors", COLD SPRING HARBOR SYMPOSIA ON QUANTITATIVE BIOLOGY, BIOLOGICAL LABORATORY, COLD SPRING HARBOR, NY, US, vol. 70, 1 January 2005 (2005-01-01), pages 399-410, XP008110855, ISSN: 0091-7451
- ABDOLLAHI AMIR ET AL: "Inhibition of alpha(v)beta3 integrin survival signaling enhances antiangiogenic and antitumor effects of radiotherapy", CLINICAL CANCER RESEARCH, THE AMERICAN ASSOCIATION FOR CANCER RESEARCH, US, vol. 11, no. 17, 1 September 2005 (2005-09-01), pages 6270-6279, XP002439489, ISSN: 1078-0432, DOI: 10.1158/1078-0432.CCR-04-1223
- BALVEEN KAUR ET AL. ONCOGENE vol. 24, 2005, pages 3632 - 3642, XP003024780
- JEONG TAE KOH ET AL. EXPERIMENTAL CELL RESEARCH vol. 294, 2004, pages 172 - 184, XP003024781
- HIROYUKI NISHIMORI ET AL. ONCOGENE vol. 15, no. 18, 1997, pages 2145 - 2150, XP000923392
- BALVEEN KAUR ET AL. AM. J. PATHOL. vol. 162, no. 1, 2003, pages 19 - 27, XP002434910
- XIAO XR ET AL. ZHONGHUA YI XUE ZA ZHI vol. 86, no. 19, 23 May 2006, pages 1342 - 1346, XP008135648

### SEQUENCE LISTING

<110> Emory University
<120> Method of Treating Abnormal Angiogenesis via the BAI family of proteins and their fragments
<130> 050508-2680
<150> US 60/936,196
   <151> 2007-06-19
<160> 5
<170> PatentIn version 3.5
<210> 1
   <211> 1584
   <212> PRT
   <213> Homo sapiens
<400> 1
   Met Arg Gly Gln Ala Ala Ala Pro Gly Pro Val Trp Ile Leu Ala Pro
   1               5                   10                  15
   Leu Leu Leu Leu Leu Leu Leu Leu Gly Arg Arg Ala Arg Ala Ala Ala
               20                  25                  30
   Gly Ala Asp Ala Gly Pro Gly Pro Glu Pro Cys Ala Thr Leu Val Gln
           35                  40                  45
   Gly Lys Phe Phe Gly Tyr Phe Ser Ala Ala Ala Val Phe Pro Ala Asn
       50                  55                  60
   Ala Ser Arg Cys Ser Trp Thr Leu Arg Asn Pro Asp Pro Arg Arg Tyr
   65                  70                  75                  80
   Thr Leu Tyr Met Lys Val Ala Lys Ala Pro Val Pro Cys Ser Gly Pro
                   85                  90                  95
   Gly Arg Val Arg Thr Tyr Gln Phe Asp Ser Phe Leu Glu Ser Thr Arg
               100                 105                 110
   Thr Tyr Leu Gly Val Glu Ser Phe Asp Glu Val Leu Arg Leu Cys Asp
           115                 120                 125
   Pro Ser Ala Pro Leu Ala Phe Leu Gln Ala Ser Lys Gln Phe Leu Gln
       130                 135                 140
   Met Arg Arg Gln Gln Pro Pro Gln His Asp Gly Leu Arg Pro Arg Ala
   145                 150                 155                 160
   Gly Pro Pro Gly Pro Thr Asp Asp Phe Ser Val Glu Tyr Leu Val Val
                   165                 170                 175
   Gly Asn Arg Asn Pro Ser Arg Ala Ala Cys Gln Met Leu Cys Arg Trp
               180                 185                 190
   Leu Asp Ala Cys Leu Ala Gly Ser Arg Ser Ser His Pro Cys Gly Ile
           195                 200                 205
   Met Gln Thr Pro Cys Ala Cys Leu Gly Gly Glu Ala Gly Gly Pro Ala
       210                 215                 220
   Ala Gly Pro Leu Ala Pro Arg Gly Asp Val Cys Leu Arg Asp Ala Val
   225                 230                 235                 240
   Ala Gly Gly Pro Glu Asn Cys Leu Thr Ser Leu Thr Gln Asp Arg Gly
                   245                 250                 255
   Gly His Gly Ala Thr Gly Gly Trp Lys Leu Trp Ser Leu Trp Gly Glu
               260                 265                 270
   Cys Thr Arg Asp Cys Gly Gly Gly Leu Gln Thr Arg Thr Arg Thr Cys
           275                 280                 285
   Leu Pro Ala Pro Gly Val Glu Gly Gly Gly Cys Glu Gly Val Leu Glu
       290                 295                 300
   Glu Gly Arg Gln Cys Asn Arg Glu Ala Cys Gly Pro Ala Gly Arg Thr
   305                 310                 315                 320
   Ser Ser Arg Ser Gln Ser Leu Arg Ser Thr Asp Ala Arg Arg Arg Glu
                   325                 330                 335
   Glu Leu Gly Asp Glu Leu Gln Gln Phe Gly Phe Pro Ala Pro Gln Thr
               340                 345                 350
   Gly Asp Pro Ala Ala Glu Glu Trp Ser Pro Trp Ser Val Cys Ser Ser
           355                 360                 365
   Thr Cys Gly Glu Gly Trp Gln Thr Arg Thr Arg Phe Cys Val Ser Ser
       370                 375                 380
   Ser Tyr Ser Thr Gln Cys Ser Gly Pro Leu Arg Glu Gln Arg Leu Cys
   385                 390                 395                 400
   Asn Asn Ser Ala Val Cys Pro Val His Gly Ala Trp Asp Glu Trp Ser
                   405                 410                 415
   Pro Trp Ser Leu Cys Ser Ser Thr Cys Gly Arg Gly Phe Arg Asp Arg
               420                 425                 430
   Thr Arg Thr Cys Arg Pro Pro Gln Phe Gly Gly Asn Pro Cys Glu Gly
           435                 440                 445
   Pro Glu Lys Gln Thr Lys Phe Cys Asn Ile Ala Leu Cys Pro Gly Arg
       450                 455                 460
   Ala Val Asp Gly Asn Trp Asn Glu Trp Ser Ser Trp Ser Ala Cys Ser
   465                 470                 475                 480
   Ala Ser Cys Ser Gln Gly Arg Gln Gln Arg Thr Arg Glu Cys Asn Gly
                   485                 490                 495
   Pro Ser Tyr Gly Gly Ala Glu Cys Gln Gly His Trp Val Glu Thr Arg
               500                 505                 510
   Asp Cys Phe Leu Gln Gln Cys Pro Val Asp Gly Lys Trp Gln Ala Trp
           515                 520                 525
   Ala Ser Trp Gly Ser Cys Ser Val Thr Cys Gly Ala Gly Ser Gln Arg
       530                 535                 540
   Arg Glu Arg Val Cys Ser Gly Pro Phe Phe Gly Gly Ala Ala Cys Gln
   545                 550                 555                 560
   Gly Pro Gln Asp Glu Tyr Arg Gln Cys Gly Thr Gln Arg Cys Pro Glu
                   565                 570                 575
   Pro His Glu Ile Cys Asp Glu Asp Asn Phe Gly Ala Val Ile Trp Lys
               580                 585                 590
   Glu Thr Pro Ala Gly Glu Val Ala Ala Val Arg Cys Pro Arg Asn Ala
           595                 600                 605
   Thr Gly Leu Ile Leu Arg Arg Cys Glu Leu Asp Glu Glu Gly Ile Ala
       610                 615                 620
   Tyr Trp Glu Pro Pro Thr Tyr Ile Arg Cys Val Ser Ile Asp Tyr Arg
   625                 630                 635                 640
   Asn Ile Gln Met Met Thr Arg Glu His Leu Ala Lys Ala Gln Arg Gly
                   645                 650                 655
   Leu Pro Gly Glu Gly Val Ser Glu Val Ile Gln Thr Leu Val Glu Ile
               660                 665                 670
   Ser Gln Asp Gly Thr Ser Tyr Ser Gly Asp Leu Leu Ser Thr Ile Asp
           675                 680                 685
   Val Leu Arg Asn Met Thr Glu Ile Phe Arg Arg Ala Tyr Tyr Ser Pro
       690                 695                 700
   Thr Pro Gly Asp Val Gln Asn Phe Val Gln Ile Leu Ser Asn Leu Leu
   705                 710                 715                 720
   Ala Glu Glu Asn Arg Asp Lys Trp Glu Glu Ala Gln Leu Ala Gly Pro
                   725                 730                 735
   Asn Ala Lys Glu Leu Phe Arg Leu Val Glu Asp Phe Val Asp Val Ile
               740                 745                 750
   Gly Phe Arg Met Lys Asp Leu Arg Asp Ala Tyr Gln Val Thr Asp Asn
           755                 760                 765
   Leu Val Leu Ser Ile His Lys Leu Pro Ala Ser Gly Ala Thr Asp Ile
       770                 775                 780
   Ser Phe Pro Met Lys Gly Trp Arg Ala Thr Gly Asp Trp Ala Lys Val
   785                 790                 795                 800
   Pro Glu Asp Arg Val Thr Val Ser Lys Ser Val Phe Ser Thr Gly Leu
                   805                 810                 815
   Thr Glu Ala Asp Glu Ala Ser Val Phe Val Val Gly Thr Val Leu Tyr
               820                 825                 830
   Arg Asn Leu Gly Ser Phe Leu Ala Leu Gln Arg Asn Thr Thr Val Leu
           835                 840                 845
   Asn Ser Lys Val Ile Ser Val Thr Val Lys Pro Pro Pro Arg Ser Leu
       850                 855                 860
   Arg Thr Pro Leu Glu Ile Glu Phe Ala His Met Tyr Asn Gly Thr Thr
   865                 870                 875                 880
   Asn Gln Thr Cys Ile Leu Trp Asp Glu Thr Asp Val Pro Ser Ser Ser
   885 890 895
   Ala Pro Pro Gln Leu Gly Pro Trp Ser Trp Arg Gly Cys Arg Thr Val
   900 905 910
   Pro Leu Asp Ala Leu Arg Thr Arg Cys Leu Cys Asp Arg Leu Ser Thr
   915 920 925
   Phe Ala Ile Leu Ala Gln Leu Ser Ala Asp Ala Asn Met Glu Lys Ala
   930 935 940
   Thr Leu Pro Ser Val Thr Leu Ile Val Gly Cys Gly Val Ser Ser Leu
   945 950 955 960
   Thr Leu Leu Met Leu Val Ile Ile Tyr Val Ser Val Trp Arg Tyr Ile
   965 970 975
   Arg Ser Glu Arg Ser Val Ile Leu Ile Asn Phe Cys Leu Ser Ile Ile
   980 985 990
   Ser Ser Asn Ala Leu Ile Leu Ile Gly Gln Thr Gln Thr Arg Asn Lys
   995 1000 1005
   Val Val Cys Thr Leu Val Ala Ala Phe Leu His Phe Phe Phe Leu
   1010 1015 1020
   Ser Ser Phe Cys Trp Val Leu Thr Glu Ala Trp Gln Ser Tyr Met
   1025 1030 1035
   Ala Val Thr Gly His Leu Arg Asn Arg Leu Ile Arg Lys Arg Phe
   1040 1045 1050
   Leu Cys Leu Gly Trp Gly Leu Pro Ala Leu Val Val Ala Ile Ser
   1055 1060 1065
   Val Gly Phe Thr Lys Ala Lys Gly Tyr Ser Thr Met Asn Tyr Cys
   1070 1075 1080
   Trp Leu Ser Leu Glu Gly Gly Leu Leu Tyr Ala Phe Val Gly Pro
   1085 1090 1095
   Ala Ala Ala Val Val Leu Val Asn Met Val Ile Gly Ile Leu Val
   1100 1105 1110
   Phe Asn Lys Leu Val Ser Lys Asp Gly Ile Thr Asp Lys Lys Leu
   1115 1120 1125
   Lys Glu  Arg Ala Gly Ala Ser  Leu Trp Ser Ser Cys  Val Val Leu
       1130                 1135                 1140
   Pro Leu  Leu Ala Leu Thr Trp  Met Ser Ala Val Leu  Ala Val Thr
       1145                 1150                 1155
   Asp Arg  Arg Ser Ala Leu Phe  Gln Ile Leu Phe Ala  Val Phe Asp
       1160                 1165                 1170
   Ser Leu  Glu Gly Phe Val Ile  Val Met Val His Cys  Ile Leu Arg
       1175                 1180                 1185
   Arg Glu  Val Gln Asp Ala Val  Lys Cys Arg Val Val  Asp Arg Gln
       1190                 1195                 1200
   Glu Glu  Gly Asn Gly Asp Ser  Gly Gly Ser Phe Gln  Asn Gly His
       1205                 1210                 1215
   Ala Gln  Leu Met Thr Asp Phe  Glu Lys Asp Val Asp  Leu Ala Cys
       1220                 1225                 1230
   Arg Ser  Val Leu Asn Lys Asp  Ile Ala Ala Cys Arg  Thr Ala Thr
       1235                 1240                 1245
   Ile Thr  Gly Thr Leu Lys Arg  Pro Ser Leu Pro Glu  Glu Glu Lys
       1250                 1255                 1260
   Leu Lys  Leu Ala His Ala Lys  Gly Pro Pro Thr Asn  Phe Asn Ser
       1265                 1270                 1275
   Leu Pro  Ala Asn Val Ser Lys  Leu His Leu His Gly  Ser Pro Arg
       1280                 1285                 1290
   Tyr Pro  Gly Gly Pro Leu Pro  Asp Phe Pro Asn His  Ser Leu Thr
       1295                 1300                 1305
   Leu Lys  Arg Asp Lys Ala Pro  Lys Ser Ser Phe Val  Gly Asp Gly
       1310                 1315                 1320
   Asp Ile  Phe Lys Lys Leu Asp  Ser Glu Leu Ser Arg  Ala Gln Glu
       1325                 1330                 1335
   Lys Ala  Leu Asp Thr Ser Tyr  Val Ile Leu Pro Thr  Ala Thr Ala
       1340                 1345                 1350
   Thr Leu Arg Pro Lys Pro Lys Glu Glu Pro Lys Tyr Ser Ile His
   1355 1360 1365
   Ile Asp Gln Met Pro Gln Thr Arg Leu Ile His Leu Ser Thr Ala
   1370 1375 1380
   Pro Glu Ala Ser Leu Pro Ala Arg Ser Pro Pro Ser Arg Gln Pro
   1385 1390 1395
   Pro Ser Gly Gly Pro Pro Glu Ala Pro Pro Ala Gln Pro Pro Pro
   1400 1405 1410
   Pro Pro Pro Pro Pro Pro Pro Pro Pro Gln Gln Pro Leu Pro Pro
   1415 1420 1425
   Pro Pro Asn Leu Glu Pro Ala Pro Pro Ser Leu Gly Asp Pro Gly
   1430 1435 1440
   Glu Pro Ala Ala His Pro Gly Pro Ser Thr Gly Pro Ser Thr Lys
   1445 1450 1455
   Asn Glu Asn Val Ala Thr Leu Ser Val Ser Ser Leu Glu Arg Arg
   1460 1465 1470
   Lys Ser Arg Tyr Ala Glu Leu Asp Phe Glu Lys Ile Met His Thr
   1475 1480 1485
   Arg Lys Arg His Gln Asp Met Phe Gln Asp Leu Asn Arg Lys Leu
   1490 1495 1500
   Gln His Ala Ala Glu Lys Asp Lys Glu Val Leu Gly Pro Asp Ser
   1505 1510 1515
   Lys Pro Glu Lys Gln Gln Thr Pro Asn Lys Arg Pro Trp Glu Ser
   1520 1525 1530
   Leu Arg Lys Ala His Gly Thr Pro Thr Trp Val Lys Lys Glu Leu
   1535 1540 1545
   Glu Pro Leu Gln Pro Ser Pro Leu Glu Leu Arg Ser Val Glu Trp
   1550 1555 1560
   Glu Arg Ser Gly Ala Thr Ile Pro Leu Val Gly Gln Asp Ile Ile
   1565 1570 1575
   Asp Leu Gln Thr Glu Val
   1580
<210> 2
   <211> 926
   <212> PRT
   <213> Homo sapiens
<400> 2
   Met Arg Gly Gln Ala Ala Ala Pro Gly Pro Val Trp Ile Leu Ala Pro
   1               5                   10                  15
   Leu Leu Leu Leu Leu Leu Leu Leu Gly Arg Arg Ala Arg Ala Ala Ala
               20                  25                  30
   Gly Ala Asp Ala Gly Pro Gly Pro Glu Pro Cys Ala Thr Leu Val Gln
           35                  40                  45
   Gly Lys Phe Phe Gly Tyr Phe Ser Ala Ala Ala Val Phe Pro Ala Asn
       50                  55                  60
   Ala Ser Arg Cys Ser Trp Thr Leu Arg Asn Pro Asp Pro Arg Arg Tyr
   65                  70                  75                  80
   Thr Leu Tyr Met Lys Val Ala Lys Ala Pro Val Pro Cys Ser Gly Pro
                   85                  90                  95
   Gly Arg Val Arg Thr Tyr Gln Phe Asp Ser Phe Leu Glu Ser Thr Arg
               100                 105                 110
   Thr Tyr Leu Gly Val Glu Ser Phe Asp Glu Val Leu Arg Leu Cys Asp
           115                 120                 125
   Pro Ser Ala Pro Leu Ala Phe Leu Gln Ala Ser Lys Gln Phe Leu Gln
       130                 135                 140
   Met Arg Arg Gln Gln Pro Pro Gln His Asp Gly Leu Arg Pro Arg Ala
   145                 150                 155                 160
   Gly Pro Pro Gly Pro Thr Asp Asp Phe Ser Val Glu Tyr Leu Val Val
                   165                 170                 175
   Gly Asn Arg Asn Pro Ser Arg Ala Ala Cys Gln Met Leu Cys Arg Trp
               180                 185                 190
   Leu Asp Ala Cys Leu Ala Gly Ser Arg Ser Ser His Pro Cys Gly Ile
           195                 200                 205
   Met Gln Thr Pro Cys Ala Cys Leu Gly Gly Glu Ala Gly Gly Pro Ala
       210                 215                 220
   Ala Gly Pro Leu Ala Pro Arg Gly Asp Val Cys Leu Arg Asp Ala Val
   225                 230                 235                 240
   Ala Gly Gly Pro Glu Asn Cys Leu Thr Ser Leu Thr Gln Asp Arg Gly
                   245                 250                 255
   Gly His Gly Ala Thr Gly Gly Trp Lys Leu Trp Ser Leu Trp Gly Glu
               260                 265                 270
   Cys Thr Arg Asp Cys Gly Gly Gly Leu Gln Thr Arg Thr Arg Thr Cys
           275                 280                 285
   Leu Pro Ala Pro Gly Val Glu Gly Gly Gly Cys Glu Gly Val Leu Glu
       290                 295                 300
   Glu Gly Arg Gln Cys Asn Arg Glu Ala Cys Gly Pro Ala Gly Arg Thr
   305                 310                 315                 320
   Ser Ser Arg Ser Gln Ser Leu Arg Ser Thr Asp Ala Arg Arg Arg Glu
                   325                 330                 335
   Glu Leu Gly Asp Glu Leu Gln Gln Phe Gly Phe Pro Ala Pro Gln Thr
               340                 345                 350
   Gly Asp Pro Ala Ala Glu Glu Trp Ser Pro Trp Ser Val Cys Ser Ser
           355                 360                 365
   Thr Cys Gly Glu Gly Trp Gln Thr Arg Thr Arg Phe Cys Val Ser Ser
       370                 375                 380
   Ser Tyr Ser Thr Gln Cys Ser Gly Pro Leu Arg Glu Gln Arg Leu Cys
   385                 390                 395                 400
   Asn Asn Ser Ala Val Cys Pro Val His Gly Ala Trp Asp Glu Trp Ser
                   405                 410                 415
   Pro Trp Ser Leu Cys Ser Ser Thr Cys Gly Arg Gly Phe Arg Asp Arg
               420                 425                 430
   Thr Arg Thr Cys Arg Pro Pro Gln Phe Gly Gly Asn Pro Cys Glu Gly
           435                 440                 445
   Pro Glu Lys Gln Thr Lys Phe Cys Asn Ile Ala Leu Cys Pro Gly Arg
       450                 455                 460
   Ala Val Asp Gly Asn Trp Asn Glu Trp Ser Ser Trp Ser Ala Cys Ser
   465                 470                 475                 480
   Ala Ser Cys Ser Gln Gly Arg Gln Gln Arg Thr Arg Glu Cys Asn Gly
                   485                 490                 495
   Pro Ser Tyr Gly Gly Ala Glu Cys Gln Gly His Trp Val Glu Thr Arg
               500                 505                 510
   Asp Cys Phe Leu Gln Gln Cys Pro Val Asp Gly Lys Trp Gln Ala Trp
           515                 520                 525
   Ala Ser Trp Gly Ser Cys Ser Val Thr Cys Gly Ala Gly Ser Gln Arg
       530                 535                 540
   Arg Glu Arg Val Cys Ser Gly Pro Phe Phe Gly Gly Ala Ala Cys Gln
   545                 550                 555                 560
   Gly Pro Gln Asp Glu Tyr Arg Gln Cys Gly Thr Gln Arg Cys Pro Glu
                   565                 570                 575
   Pro His Glu Ile Cys Asp Glu Asp Asn Phe Gly Ala Val Ile Trp Lys
               580                 585                 590
   Glu Thr Pro Ala Gly Glu Val Ala Ala Val Arg Cys Pro Arg Asn Ala
           595                 600                 605
   Thr Gly Leu Ile Leu Arg Arg Cys Glu Leu Asp Glu Glu Gly Ile Ala
       610                 615                 620
   Tyr Trp Glu Pro Pro Thr Tyr Ile Arg Cys Val Ser Ile Asp Tyr Arg
   625                 630                 635                 640
   Asn Ile Gln Met Met Thr Arg Glu His Leu Ala Lys Ala Gln Arg Gly
                   645                 650                 655
   Leu Pro Gly Glu Gly Val Ser Glu Val Ile Gln Thr Leu Val Glu Ile
               660                 665                 670
   Ser Gln Asp Gly Thr Ser Tyr Ser Gly Asp Leu Leu Ser Thr Ile Asp
           675                 680                 685
   Val Leu Arg Asn Met Thr Glu Ile Phe Arg Arg Ala Tyr Tyr Ser Pro
   690 695 700
   Thr Pro Gly Asp Val Gln Asn Phe Val Gln Ile Leu Ser Asn Leu Leu
   705 710 715 720
   Ala Glu Glu Asn Arg Asp Lys Trp Glu Glu Ala Gln Leu Ala Gly Pro
   725 730 735
   Asn Ala Lys Glu Leu Phe Arg Leu Val Glu Asp Phe Val Asp Val Ile
   740 745 750
   Gly Phe Arg Met Lys Asp Leu Arg Asp Ala Tyr Gln Val Thr Asp Asn
   755 760 765
   Leu Val Leu Ser Ile His Lys Leu Pro Ala Ser Gly Ala Thr Asp Ile
   770 775 780
   Ser Phe Pro Met Lys Gly Trp Arg Ala Thr Gly Asp Trp Ala Lys Val
   785 790 795 800
   Pro Glu Asp Arg Val Thr Val Ser Lys Ser Val Phe Ser Thr Gly Leu
   805 810 815
   Thr Glu Ala Asp Glu Ala Ser Val Phe Val Val Gly Thr Val Leu Tyr
   820 825 830
   Arg Asn Leu Gly Ser Phe Leu Ala Leu Gln Arg Asn Thr Thr Val Leu
   835 840 845
   Asn Ser Lys Val Ile Ser Val Thr Val Lys Pro Pro Pro Arg Ser Leu
   850 855 860
   Arg Thr Pro Leu Glu Ile Glu Phe Ala His Met Tyr Asn Gly Thr Thr
   865 870 875 880
   Asn Gln Thr Cys Ile Leu Trp Asp Glu Thr Asp Val Pro Ser Ser Ser
   885 890 895
   Ala Pro Pro Gln Leu Gly Pro Trp Ser Trp Arg Gly Cys Arg Thr Val
   900 905 910
   Pro Leu Asp Ala Leu Arg Thr Arg Cys Leu Cys Asp Arg Leu
   915 920 925
<210> 3
   <211> 328
   <212> PRT
   <213> Homo sapiens
<400> 3
   Met Arg Gly Gln Ala Ala Ala Pro Gly Pro Val Trp Ile Leu Ala Pro
   1               5                   10                  15
   Leu Leu Leu Leu Leu Leu Leu Leu Gly Arg Arg Ala Arg Ala Ala Ala
               20                  25                  30
   Gly Ala Asp Ala Gly Pro Gly Pro Glu Pro Cys Ala Thr Leu Val Gln
           35                  40                  45
   Gly Lys Phe Phe Gly Tyr Phe Ser Ala Ala Ala Val Phe Pro Ala Asn
       50                  55                  60
   Ala Ser Arg Cys Ser Trp Thr Leu Arg Asn Pro Asp Pro Arg Arg Tyr
   65                  70                  75                  80
   Thr Leu Tyr Met Lys Val Ala Lys Ala Pro Val Pro Cys Ser Gly Pro
                   85                  90                  95
   Gly Arg Val Arg Thr Tyr Gln Phe Asp Ser Phe Leu Glu Ser Thr Arg
               100                 105                 110
   Thr Tyr Leu Gly Val Glu Ser Phe Asp Glu Val Leu Arg Leu Cys Asp
           115                 120                 125
   Pro Ser Ala Pro Leu Ala Phe Leu Gln Ala Ser Lys Gln Phe Leu Gln
       130                 135                 140
   Met Arg Arg Gln Gln Pro Pro Gln His Asp Gly Leu Arg Pro Arg Ala
   145                 150                 155                 160
   Gly Pro Pro Gly Pro Thr Asp Asp Phe Ser Val Glu Tyr Leu Val Val
                   165                 170                 175
   Gly Asn Arg Asn Pro Ser Arg Ala Ala Cys Gln Met Leu Cys Arg Trp
               180                 185                 190
   Leu Asp Ala Cys Leu Ala Gly Ser Arg Ser Ser His Pro Cys Gly Ile
           195                 200                 205
   Met Gln Thr Pro Cys Ala Cys Leu Gly Gly Glu Ala Gly Gly Pro Ala
       210                 215                 220
   Ala Gly Pro Leu Ala Pro Arg Gly Asp Val Cys Leu Arg Asp Ala Val
   225                 230                 235                 240
   Ala Gly Gly Pro Glu Asn Cys Leu Thr Ser Leu Thr Gln Asp Arg Gly
                   245                 250                 255
   Gly His Gly Ala Thr Gly Gly Trp Lys Leu Trp Ser Leu Trp Gly Glu
               260                 265                 270
   Cys Thr Arg Asp Cys Gly Gly Gly Leu Gln Thr Arg Thr Arg Thr Cys
           275                 280                 285
   Leu Pro Ala Pro Gly Val Glu Gly Gly Gly Cys Glu Gly Val Leu Glu
       290                 295                 300
   Glu Gly Arg Gln Cys Asn Arg Glu Ala Cys Gly Pro Ala Gly Arg Thr
   305                 310                 315                 320
   Ser Ser Arg Ser Gln Ser Leu Arg
                   325
<210> 4
   <211> 894
   <212> PRT
   <213> Homo sapiens
<400> 4
   Gly Ala Asp Ala Gly Pro Gly Pro Glu Pro Cys Ala Thr Leu Val Gln
   1               5                   10                  15
   Gly Lys Phe Phe Gly Tyr Phe Ser Ala Ala Ala Val Phe Pro Ala Asn
               20                  25                  30
   Ala Ser Arg Cys Ser Trp Thr Leu Arg Asn Pro Asp Pro Arg Arg Tyr
           35                  40                  45
   Thr Leu Tyr Met Lys Val Ala Lys Ala Pro Val Pro Cys Ser Gly Pro
       50                  55                  60
   Gly Arg Val Arg Thr Tyr Gln Phe Asp Ser Phe Leu Glu Ser Thr Arg
   65                  70                  75                  80
   Thr Tyr Leu Gly Val Glu Ser Phe Asp Glu Val Leu Arg Leu Cys Asp
                   85                  90                  95
   Pro Ser Ala Pro Leu Ala Phe Leu Gln Ala Ser Lys Gln Phe Leu Gln
               100                 105                 110
   Met Arg Arg Gln Gln Pro Pro Gln His Asp Gly Leu Arg Pro Arg Ala
           115                 120                 125
   Gly Pro Pro Gly Pro Thr Asp Asp Phe Ser Val Glu Tyr Leu Val Val
       130                 135                 140
   Gly Asn Arg Asn Pro Ser Arg Ala Ala Cys Gln Met Leu Cys Arg Trp
   145                 150                 155                 160
   Leu Asp Ala Cys Leu Ala Gly Ser Arg Ser Ser His Pro Cys Gly Ile
                   165                 170                 175
   Met Gln Thr Pro Cys Ala Cys Leu Gly Gly Glu Ala Gly Gly Pro Ala
               180                 185                 190
   Ala Gly Pro Leu Ala Pro Arg Gly Asp Val Cys Leu Arg Asp Ala Val
           195                 200                 205
   Ala Gly Gly Pro Glu Asn Cys Leu Thr Ser Leu Thr Gln Asp Arg Gly
       210                 215                 220
   Gly His Gly Ala Thr Gly Gly Trp Lys Leu Trp Ser Leu Trp Gly Glu
   225                 230                 235                 240
   Cys Thr Arg Asp Cys Gly Gly Gly Leu Gln Thr Arg Thr Arg Thr Cys
                   245                 250                 255
   Leu Pro Ala Pro Gly Val Glu Gly Gly Gly Cys Glu Gly Val Leu Glu
               260                 265                 270
   Glu Gly Arg Gln Cys Asn Arg Glu Ala Cys Gly Pro Ala Gly Arg Thr
           275                 280                 285
   Ser Ser Arg Ser Gln Ser Leu Arg Ser Thr Asp Ala Arg Arg Arg Glu
       290                 295                 300
   Glu Leu Gly Asp Glu Leu Gln Gln Phe Gly Phe Pro Ala Pro Gln Thr
   305                 310                 315                 320
   Gly Asp Pro Ala Ala Glu Glu Trp Ser Pro Trp Ser Val Cys Ser Ser
                   325                 330                 335
   Thr Cys Gly Glu Gly Trp Gln Thr Arg Thr Arg Phe Cys Val Ser Ser
               340                 345                 350
   Ser Tyr Ser Thr Gln Cys Ser Gly Pro Leu Arg Glu Gln Arg Leu Cys
           355                 360                 365
   Asn Asn Ser Ala Val Cys Pro Val His Gly Ala Trp Asp Glu Trp Ser
       370                 375                 380
   Pro Trp Ser Leu Cys Ser Ser Thr Cys Gly Arg Gly Phe Arg Asp Arg
   385                 390                 395                 400
   Thr Arg Thr Cys Arg Pro Pro Gln Phe Gly Gly Asn Pro Cys Glu Gly
                   405                 410                 415
   Pro Glu Lys Gln Thr Lys Phe Cys Asn Ile Ala Leu Cys Pro Gly Arg
               420                 425                 430
   Ala Val Asp Gly Asn Trp Asn Glu Trp Ser Ser Trp Ser Ala Cys Ser
           435                 440                 445
   Ala Ser Cys Ser Gln Gly Arg Gln Gln Arg Thr Arg Glu Cys Asn Gly
       450                 455                 460
   Pro Ser Tyr Gly Gly Ala Glu Cys Gln Gly His Trp Val Glu Thr Arg
   465                 470                 475                 480
   Asp Cys Phe Leu Gln Gln Cys Pro Val Asp Gly Lys Trp Gln Ala Trp
                   485                 490                 495
   Ala Ser Trp Gly Ser Cys Ser Val Thr Cys Gly Ala Gly Ser Gln Arg
               500                 505                 510
   Arg Glu Arg Val Cys Ser Gly Pro Phe Phe Gly Gly Ala Ala Cys Gln
           515                 520                 525
   Gly Pro Gln Asp Glu Tyr Arg Gln Cys Gly Thr Gln Arg Cys Pro Glu
       530                 535                 540
   Pro His Glu Ile Cys Asp Glu Asp Asn Phe Gly Ala Val Ile Trp Lys
   545                 550                 555                 560
   Glu Thr Pro Ala Gly Glu Val Ala Ala Val Arg Cys Pro Arg Asn Ala
                   565                 570                 575
   Thr Gly Leu Ile Leu Arg Arg Cys Glu Leu Asp Glu Glu Gly Ile Ala
               580                 585                 590
   Tyr Trp Glu Pro Pro Thr Tyr Ile Arg Cys Val Ser Ile Asp Tyr Arg
           595                 600                 605
   Asn Ile Gln Met Met Thr Arg Glu His Leu Ala Lys Ala Gln Arg Gly
       610                 615                 620
   Leu Pro Gly Glu Gly Val Ser Glu Val Ile Gln Thr Leu Val Glu Ile
   625                 630                 635                 640
   Ser Gln Asp Gly Thr Ser Tyr Ser Gly Asp Leu Leu Ser Thr Ile Asp
                   645                 650                 655
   Val Leu Arg Asn Met Thr Glu Ile Phe Arg Arg Ala Tyr Tyr Ser Pro
               660                 665                 670
   Thr Pro Gly Asp Val Gln Asn Phe Val Gln Ile Leu Ser Asn Leu Leu
           675                 680                 685
   Ala Glu Glu Asn Arg Asp Lys Trp Glu Glu Ala Gln Leu Ala Gly Pro
       690                 695                 700
   Asn Ala Lys Glu Leu Phe Arg Leu Val Glu Asp Phe Val Asp Val Ile
   705                 710                 715                 720
   Gly Phe Arg Met Lys Asp Leu Arg Asp Ala Tyr Gln Val Thr Asp Asn
                   725                 730                 735
   Leu Val Leu Ser Ile His Lys Leu Pro Ala Ser Gly Ala Thr Asp Ile
               740                 745                 750
   Ser Phe Pro Met Lys Gly Trp Arg Ala Thr Gly Asp Trp Ala Lys Val
           755                 760                 765
   Pro Glu Asp Arg Val Thr Val Ser Lys Ser Val Phe Ser Thr Gly Leu
       770                 775                 780
   Thr Glu Ala Asp Glu Ala Ser Val Phe Val Val Gly Thr Val Leu Tyr
   785                 790                 795                 800
   Arg Asn Leu Gly Ser Phe Leu Ala Leu Gln Arg Asn Thr Thr Val Leu
                   805                 810                 815
   Asn Ser Lys Val Ile Ser Val Thr Val Lys Pro Pro Pro Arg Ser Leu
               820                 825                 830
   Arg Thr Pro Leu Glu Ile Glu Phe Ala His Met Tyr Asn Gly Thr Thr
           835                 840                 845
   Asn Gln Thr Cys Ile Leu Trp Asp Glu Thr Asp Val Pro Ser Ser Ser
       850                 855                 860
   Ala Pro Pro Gln Leu Gly Pro Trp Ser Trp Arg Gly Cys Arg Thr Val
   865                 870                 875                 880
   Pro Leu Asp Ala Leu Arg Thr Arg Cys Leu Cys Asp Arg Leu
                   885                 890
<210> 5
   <211> 296
   <212> PRT
   <213> Homo sapiens
<400> 5
   Gly Ala Asp Ala Gly Pro Gly Pro Glu Pro Cys Ala Thr Leu Val Gln
   1               5                   10                  15
   Gly Lys Phe Phe Gly Tyr Phe Ser Ala Ala Ala Val Phe Pro Ala Asn
               20                  25                  30
   Ala Ser Arg Cys Ser Trp Thr Leu Arg Asn Pro Asp Pro Arg Arg Tyr
           35                  40                  45
   Thr Leu Tyr Met Lys Val Ala Lys Ala Pro Val Pro Cys Ser Gly Pro
       50                  55                  60
   Gly Arg Val Arg Thr Tyr Gln Phe Asp Ser Phe Leu Glu Ser Thr Arg
   65                  70                  75                  80
   Thr Tyr Leu Gly Val Glu Ser Phe Asp Glu Val Leu Arg Leu Cys Asp
                   85                  90                  95
   Pro Ser Ala Pro Leu Ala Phe Leu Gln Ala Ser Lys Gln Phe Leu Gln
               100                 105                 110
   Met Arg Arg Gln Gln Pro Pro Gln His Asp Gly Leu Arg Pro Arg Ala
           115                 120                 125
   Gly Pro Pro Gly Pro Thr Asp Asp Phe Ser Val Glu Tyr Leu Val Val
       130                 135                 140
   Gly Asn Arg Asn Pro Ser Arg Ala Ala Cys Gln Met Leu Cys Arg Trp
   145                 150                 155                 160
   Leu Asp Ala Cys Leu Ala Gly Ser Arg Ser Ser His Pro Cys Gly Ile
                   165                 170                 175
   Met Gln Thr Pro Cys Ala Cys Leu Gly Gly Glu Ala Gly Gly Pro Ala
               180                 185                 190
   Ala Gly Pro Leu Ala Pro Arg Gly Asp Val Cys Leu Arg Asp Ala Val
           195                 200                 205
   Ala Gly Gly Pro Glu Asn Cys Leu Thr Ser Leu Thr Gln Asp Arg Gly
       210                 215                 220
   Gly His Gly Ala Thr Gly Gly Trp Lys Leu Trp Ser Leu Trp Gly Glu
   225                 230                 235                 240
   Cys Thr Arg Asp Cys Gly Gly Gly Leu Gln Thr Arg Thr Arg Thr Cys
                   245                 250                 255
   Leu Pro Ala Pro Gly Val Glu Gly Gly Gly Cys Glu Gly Val Leu Glu
               260                 265                 270
   Glu Gly Arg Gln Cys Asn Arg Glu Ala Cys Gly Pro Ala Gly Arg Thr
           275                 280                 285
   Ser Ser Arg Ser Gln Ser Leu Arg
       290                 295

## Description

### CROSS-REFERENCE TO RELATED APPLICATION

This application claims priority to U.S. provisional application entitled "A Method of Treating Abnormal Angiogenesis via the BAI Family of Proteins and their Protein Fragments," having serial number 60/936,196 filed on June 19, 2007.

### STATEMENT REGARDING FEDERALLY SPONSORED

### RESEARCH OR DEVELOPMENT

This invention(s) was made in part with government support under Grant Nos.: CA86335, HL67839, and NS056203. The government has certain rights in the disclosure(s).

### FIELD OF THE DISCLOSURE

This disclosure relates to the Brain Angiogenesis Inhibitor BAI1, its cleavage fragments Vasculostatin 120 (Vstat120) and Vasculostatin 40 (Vstat40), and homologs thereof. This disclosure further relates to the use of these polypeptides as anti-angiogenic and anti-tumorigenic agents.

### BACKGROUND

Angiogenesis, the development of new blood vessels, plays crucial roles both in embryonic development and in various physiological or pathological processes. These include wound healing, cardiovascular diseases such as atherosclerosis, aortic stenosis and ischemic heart disease, arthritis, psoriasis, diabetes, chronic inflammatory diseases, peripheral vascular diseases, various forms of blindness such as macular degeneration and cancer. With cancers, the realization that tumors need to establish a vascular supply in order to provide nutrient support as well as providing a waste pathway, has excited much interest in the possibility that inhibition of angiogenesis offers a *novel* way in which to inhibit tumor growth. To obtain blood supply for their growth, tumor cells are potently angiogenic and attract new vessels through increased secretion of inducers (growth factors) and decreased production of endogenous negative regulators. This balance of factors is usually tightly controlled with new vessel growth suppressed under normal physiologic conditions. A change in this balance is sometimes referred to as the "angiogenic switch". The discovery of new pro- and anti-angiogenic factors and the ability to regulate their expression endogenously or administer them exogenously has potential implications to multiple conditions, and presents a unique avenue for the development of therapeutics.

The process of angiogenesis is tightly regulated in normal adult tissues by maintaining a delicate balance between pro-angiogenic and anti-angiogenic factors. In neoplasia, this balance is tilted in favor of new blood-vessel development, thereby increasing its vascular supply and promoting growth and metastasis (Folkman, J. N. Engl. J. Med., 1971; 285: 1182-1186; Wang et al., Brain Pathol., 2005; 15: 318-326). The production of pro-angiogenic molecules, such as Vascular Endothelial Growth factor (VEGF) and IL-8 is increased, and the expression of anti-angiogenic factors, such as thrombospondin-1 (TSP-1) is reduced (Tenan et al., J. Exp. Med., 2000; 191: 1789-1798; 4. Desbaillets et al., J. Exp. Med., 1997; 186: 1201-1212). These secondary angiogenesis regulatory events are the consequences of loss of tumor suppressors such as p53 and PTEN or gain of oncogene expression such as EGFR. Arresting angiogenesis in combination with other agents is currently being exploited as an effective new therapeutic modality for cancer (Batchelor et al., Cancer Cell, 2007; 11: 83-95; Kurozumi et al., J. Natl. Cancer Inst., 2007; 99: 1768-1781). Little is known about how physiological angiogenesis is regulated in the brain and how it becomes co-opted during brain tumor development.

Gliomas are the most common primary tumor of the central nervous system. Glioblastoma multiforme (GBM), the most aggressive form of malignant astrocytoma (WHO grade IV) is characterized pathologically by a highly abnormal vasculature (Brat et al., Cancer Res., 2004; 64: 920-927). During astrocytoma progression from low to high grade, increase in vessel density precedes malignant progression as well as an accumulation of genetic defects. The two genetic alterations that coincide with transition to WHO grade IV GBM are the loss of the *PTEN* tumor suppressor gene and the amplification of the *EGFR* proto-oncogene (Li et al., Science, 1997; 275: 1943-1947; Smith et al., J Natl Cancer Inst, 2001; 93: 1246-1256). Apart from gene amplification and receptor over-expression, the EGFR gene is also frequently mutated in GBM. The most common of these mutations results in a truncated ligand-independent EGFRvIII with constitutive activity (Ekstrand et al., Proc. Natl. Acad. Sci. U.S.A., 1992; 89: 4309-4313; Libermann et al., Nature, 1985; 313: 144-147). Importantly, both these events are known to increase the angiogenic phenotype of glioma cells.

The brain angiogenesis inhibitor 1 (BAI1) is a member of the adhesion subfamily of G protein-coupled receptors (GPCRs), thought to be involved in cell-cell and cell-matrix interactions (Shiratsuchi et al., Biochem. Biophys. Res. Commun., 1998;247: 597-604; Nishimori et al., Oncogene, 1997;15: 2145-2150). Its expression is reduced in malignant gliomas, pulmonary adenocarcinoma, pancreatic and gastric cancers, but present in the corresponding normal tissue with by far the most abundant expression in the brain (Kaur et al., Am. J. Pathol., 2003; 162: 19-27; Hatanaka et al., Int. J. Mol. Med., 2000;5: 181-183; Fukushima et al., Int. J. Oncol., 1998; 13: 967-970.).

Brain angiogenesis inhibitor 1 (BAI1), may contribute to the regulation of the "angiogenic switch" and its loss of expression appears important to the progression of gliomas (Kaur et al, 2003 Am. J. Pathol. 162: 19-27). BAI1 is a 1584 amino acid transmembrane protein structured similarly to a class B seven transmembrane G-protein coupled receptor. It has both a 45 kDa intracellular domain whose precise role is unknown, and a large 120 kDa extracellular domain. This domain contains two important areas, an Arg-Gly-Asp (RGD) integrin-binding motif, as well as five thrombospondin type 1 repeats (TSRs). The RGD integrin-binding motif confers it with the ability to interact with cell surface integrins and may influence cell migration and intracellular growth factor signaling, while the presence of TSRs indicates possible anti-angiogenic functions.

Re-expression of BAI1 in tumor cells that have lost its expression has been shown to result in slow growing tumors with reduced vessel density, suggesting an anti-angiogenic function (Kudo et al., Oncol. Rep., 2007; 18: 785-791; Kang et al., Cancer Gene Ther, 2006; 13: 385-392). Vasculostatin is a naturally occurring 120 kDa fragment (Vstat120) of BAI1 (Kaur et al., Oncogene, 2005; 24: 3632-3642) and is released from BAI1 by proteolytic cleavage at a consensus GPS site located close to the junction with the plasma membrane. Vstat120 contains the Arginine-Glycine-Aspartate (RGD) integrin recognition motif and 5 thrombospondin type 1 repeats (TSRs).

Tumor growth as well as its response to targeted treatments is affected by its location and microenvironment (Blouw et al., Cancer Cell, 2003; 4: 133-146). Vstat120 can suppress the growth of glial tumors in a subcutaneous mouse xenograft model (Kaur et al., Oncogene, 2005; 24: 3632-3642). However, despite its primarily brain-specific expression, an effect of Vstat120 on the growth of intracerebral tumors has not yet been reported.

Gliomas arising from glial cells are the most common primary tumor type occurring within the central nervous system. Of these, anaplastic astrocytomas and Glioblastoma Multiforme (GBM) are the most common and aggressive forms. While advances in detection, surgery, chemotherapy and radiation have improved the outcome of many cancer types, the median survival after initial diagnosis with GBM remains low, approximately 50 weeks, and survival beyond 3 years at 2%. This leaves the development and identification of new therapies and new therapeutic targets as one possible avenue for improving treatment.

### SUMMARY

According to claim 1, the present invention relates, in at least one embodiment, to a pharmaceutical composition comprising a polypeptide comprising an integrin binding domain and one thrombospondin type 1 repeat, wherein the amino acid sequence of the polypeptide is selected from the group of SEQ ID NO.: 3, SEQ ID NO.: 5, and conservative variants thereof having at least 80% identity to SEQ ID NO.: 3 or SEQ ID NO.: 5, whereby the polypeptide binds CD36 via the thrombospondin type 1 repeat and inhibits angiogenesis.

According to claim 2, the polypeptide has the amino acid sequence of SEQ ID NO.: 3.

Embodiments of the present disclosure encompasses the protein BAI1 of the BAI family, and its two cleavage products, the *novel* protein fragments Vstat120 and Vstat40. The present disclosure also describes the use of Vstat120 and Vstat40 as an anti-angiogenic and anti-tumorigenic therapy for gliomas as well as other types of cancer and conditions involving aberrant angiogenesis.

One aspect of the present disclosure is a polypeptide, wherein the amino acid sequence of the polypeptide has an amino acid sequence selected from the group consisting of SEQ ID NOS.: 3 and 4, and conservative variants thereof, and wherein the polypeptide comprises an integrin binding domain and a thrombospondin type 1 repeat.

In one embodiment of this aspect of the disclosure, the polypeptide may have the amino acid sequence according to SEQ ID NO.: 3. In another embodiment of this aspect of the disclosure, the polypeptide may have the amino acid sequence according to SEQ ID NO.: 4.

In another embodiment of the disclosure, the polypeptide may be isolated from a cell culture, wherein the cell culture may be comprised of animal or human cells comprising a heterologous nucleic acid encoding the polypeptide, and wherein the heterologous nucleic acid may be an expression vector comprising a region encoding the polypeptide operably linked to a gene expression regulatory region.

In various aspects of the disclosure, the expression vector may be selected from the group consisting of: a plasmid vector, a viral vector, and an artificial chromosome, and wherein the expression vector optionally is incorporated into the genomic DNA of the animal or human cells.

Preferably, the expression vector comprising a heterologous nucleic acid encoding a polypeptide that has the amino acid sequence of SEQ ID NO.: 3, SEQ ID NO.: 5, or conservative variants thereof having at least 80% identity to SEQ ID NO.: 3 or SEQ ID NO.: 5, wherein the polypeptide consists of an integrin binding domain and one thrombospondin type 1 repeat, and whereby the polypeptide binds CD36 via the thrombospondin type 1 repeat and inhibits angiogenesis.

Another embodiment of the disclosure provides methods of preparing a polypeptide, comprising: providing a first polypeptide, wherein the first polypeptide is BAI1 having an amino acid sequence according to SEQ ID NO.: 1, or an extracellular fragment thereof, wherein the extracellular fragment has a sequence selected from the group consisting of: SEQ ID NO.: 2, SEQ ID NO.: 4 and conservative variants thereof having at least 80% identity to SEQ ID NO.: 2 or SEQ ID NO.: 4; and contacting the first polypeptide with furin thereby forming a second polypeptide comprising an integrin binding domain and at least one thrombospondin type 1 repeat..

The first polypeptide may be according to SEQ ID NO.: 1, and the second polypeptide has an amino acid sequence selected from the group consisting of: SEQ ID NOS.: 2, 3, 4, 5, and conservative variants thereof. In one embodiment of the disclosure, the first polypeptide may have the amino acid sequence according to SEQ ID NO.: 2, or conservative variants thereof having at least 80% identity to SEQ ID NO.: 2, and the second polypeptide may have the amino acid sequence according to SEQ ID NO.: 3 or conservative variants thereof having at least 80% identity to SEQ ID NO.: 3. In another embodiment, the first polypeptide may have the amino acid sequence according to SEQ ID NO.: 4, or conservative variants thereof having at least 80% identity to SEQ ID NO.: 4, and the second polypeptide may have the amino acid sequence according to SEQ ID NO.: 5, or conservative variants thereof having at least 80% identity to SEQ ID NO.: 5. The method may further comprise isolating the second polypeptide.

Another aspect of the present disclosure is an expression vector selected from the group consisting of: a plasmid vector, a viral vector, and an artificial chromosome, and wherein the expression vector comprises a heterologous nucleic acid encoding a polypeptide having an amino acid sequence selected from the group consisting of SEQ ID NOS.: 3 and 4, and conservative variants thereof, and wherein the polypeptide comprises an integrin binding domain and a thrombospondin type 1 repeat. In embodiments of this aspect of the disclosure, the polypeptide encoded by the heterologous nucleic acid has the amino acid sequence according to SEQ ID NO.: 3. In other embodiments of the disclosure, the polypeptide encoded by the heterologous nucleic acid has the amino acid sequence according to SEQ ID NO.: 4.

Yet another aspect of the disclosure are methods of inhibiting the proliferation of endothelial cells comprising: contacting a population of endothelial cells with a polypeptide having an amino acid sequence derived from that of the protein BAI1 (SEQ ID NO.: 1), wherein the amino acid sequence of the polypeptide has an amino acid sequence selected from the group consisting of: SEQ ID NOS.: 3 and 5, or conservative variants thereof, and wherein the cleavage product comprises an integrin binding domain and a thrombospondin type 1 repeat, whereby contacting the endothelial cells with the polypeptide inhibits the proliferation of the endothelial cells.

In this aspect of the disclosure, the population of endothelial cells may be in an animal or human, and the method may further comprise systemically administering the polypeptide to the animal or the human. The method may further comprise directly delivering the polypeptide to a population of cells in the animal or the human.

Another aspect of the disclosure provides methods of inhibiting angiogenesis comprising: contacting a population of endothelial cells with a polypeptide, wherein the polypeptide has an amino acid sequence selected from the group consisting of: SEQ ID NOS.: 2, 3, 4, 5, or conservative variants thereof, and wherein the polypeptide comprises an integrin binding domain and at least one thrombospondin type 1 repeat, whereby contacting the endothelial cells with the polypeptide inhibits the proliferation of the endothelial cells thereby inhibiting angiogenesis. The method may further comprise delivering the polypeptide to an animal or human, whereby angiogenesis is inhibited in the animal or human, and the polypeptide may be delivered to an animal or human as a bolus or as a sustained delivery.

In this aspect of the invention, the polypeptide may be delivered to an animal or human by administering thereto a pharmaceutically acceptable composition comprising a nucleic acid vector incorporating therein a heterologous nucleic acid sequence encoding a polypeptide having an amino acid sequence selected from the group consisting of: SEQ ID NOS.: 2, 3, 4, 5, or conservative variants thereof; and expressing the heterologous nucleic acid sequence, thereby delivering the polypeptide to the endothelial cells.

In various aspects of this method of the disclosure, the nucleic acid vector may be a plasmid vector or a viral vector.

In these aspects of this method of the disclosure, the pathological condition may be a tumor, a wound, or age-related macular degeneration.

Still another aspect of the disclosure provides methods of inhibiting the formation of a tumor in an animal or human, wherein the tumor is sustained or disseminated by angiogenesis, comprising: contacting a developing tumor in an animal or human with a polypeptide derived from the protein BAI1 (SEQ ID NO.: 1), wherein the amino acid sequence of the polypeptide may have an amino acid sequence selected from the group consisting of: SEQ ID NOS.: 2, 3, 4, 5, and wherein the polypeptide comprises an integrin binding domain and at least one thrombospondin type 1 repeat, whereby contacting the tumor with the polypeptide inhibits angiogenesis by binding to the CD36 receptor on endothelial cells, thereby inhibiting the formation of the tumor such as a tumor of the brain, including a glioma.

In this aspect of the disclosure, the method may further comprise directly delivering the polypeptide to the tumor of the brain by injection into the tumor tissue or injection into a blood vessel leading into the tumor.

Still another aspect of the disclosure provides a pharmaceutical composition comprising an isolated polypeptide derived from the protein BAI1 (SEQ ID NO.: 1), wherein the amino acid sequence of the polypeptide may have at least 80% similarity with a sequence selected from the group consisting of: SEQ ID NOS.: 2, 3, 4, 5, or conservative variants thereof, and comprises an integrin binding domain and at least one thrombospondin type 1 repeat, and a pharmaceutically acceptable carrier.

### BRIEF DESCRIPTION OF THE DRAWINGS

Figs. 1A -1C illustrate that the expression of Vstat120 enhances the survival of rats implanted with U87MG glioma cells in the brain.
Fig. 1A illustrates Western blot analysis of cell lysates from U87MG parental cells, and U87MG derived clones stably transfected with Vstat120 cDNA (U14 and U18).
Fig. 1B is a graph that illustrates the *in vitro* proliferation rates of U87MG cells, and clones U14 and U18 as determined by the crystal violet assay.
Fig. 1C is a graph that illustrates the intracranial tumorigenicity assay for U87MG and Vstat120 expressing clones, U14 and U18. 1 x 10⁶ cells were implanted stereotactically in the brain of athymic nude rats. Kaplan-Meyer curves of rats implanted with cells expressing Vstat120 showed a significant improvement in their survival compared to the control parental U87MG cells (p<0.05).
Figs. 2A-2D illustrate that Vstat120 expression suppresses subcutaneous and intracranial tumor growth of U87 ΔEGFR cells despite the pro-angiogenic stimulus provided by EGFRvIII.

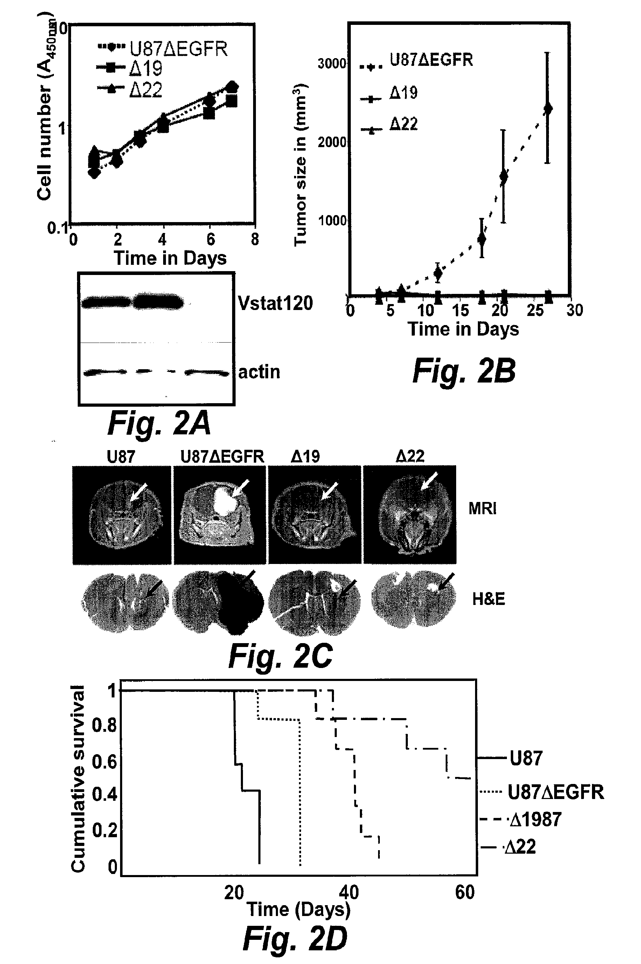

Fig. 2A is a graph that illustrates the characterization of U87ΔEGFR and Vstat120 expressing clones Δ19 and Δ22. *In vitro* proliferation rates of U87 ΔEGFR cells and Vstat120 expressing clones Δ19 and Δ22 were measured using the crystal violet assay. Expression of Vstat120 did not alter the *in vitro* proliferation rates of these cells. Bottom Panel shows western blot analysis of cell lysates from U87ΔEGFR cells (lane 3) and derived clones Δ19 and Δ22 (lane 1 and 2 respectively), which stably express Vstat120.
Fig. 2B is a graph that illustrates U87 ΔEGFR and cells stably expressing Vstat120 (Δ19 and Δ22) were injected subcutaneously into mice (n=6) and the tumor volume for the indicated clones was plotted as a function of time, with tumor growth decrease of clones expressing Vstat120.
   The upper panels of Fig. 2C show representative images of the MRI scans of individual rat brains. The presence of glioma is detected through the bright areas of contrast enhancement from the gadolinium agent (white arrow). Note the small tumor in U87MG cells, large tumor in U87ΔEGFR cells and barely detectable minimal tumors in clones Δ22 and Δ19. The lower panel shows corresponding histopathological brain sections stained with H&E. Tumor growth is visible as a dark blue area (black arrow).
Fig. 2D is a graph that illustrates the Kaplan Meier survival curve of rats implanted with U87MG, U87ΔEGFR and Vstat120 expressing clones, Δ19 and Δ22. 1 x 10⁶ cells were implanted stereotactically in the brain of athymic *nu*/*nu* rats. Rats implanted with U87ΔEGFR cells had the shortest survival time due to the very angiogenic and aggressive nature of these tumors. Vstat120 expressing clones Δ19 and Δ22, showed a significant improvement in their survival compared to the U87ΔEGFR and control parental U87MG cells (p<0.05).

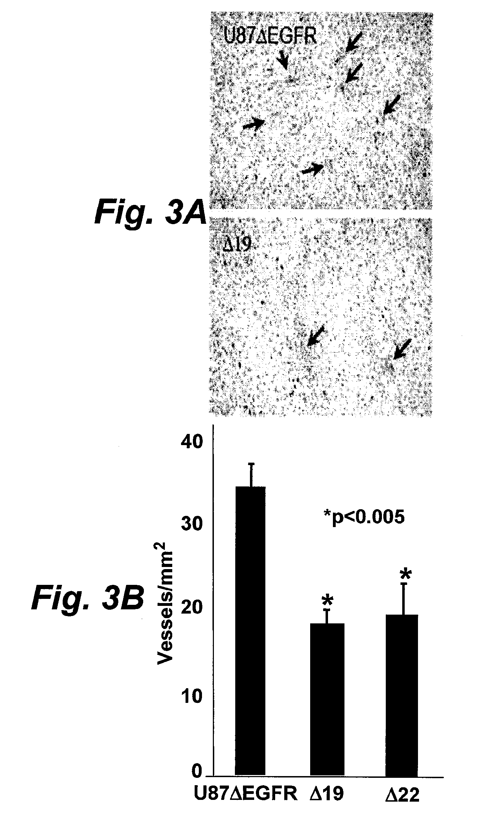

Figs. 3A and 3B illustrate that Vstat120 reduces vascular density of U87ΔEGFR tumors grown intracranially.
Fig. 3A shows representative pictures of the immunohistochemistry for von Willebrand factor in tumor sections derived from U87ΔEGFR or Vstat120 expressing clone (Δ19) are shown. Brown staining indicates endothelial cells lining capillaries (arrows).
Fig. 3B is a graph that illustrates vessel densities in U87-EGFR and Vstat120-expressing clones (Δ19 and Δ22). Vstat120-expressing tumors showed significantly lower vessel density than parental tumors. Vessel densities are expressed as mean +/- SEM. * p<0.005

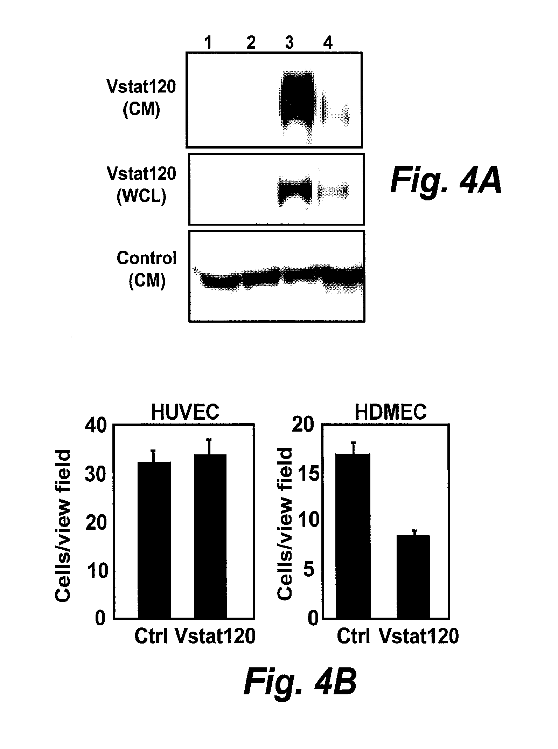

Figs. 4A-4D illustrate that Vstat120 inhibits endothelial cell migration in a CD36-dependent fashion.
Fig. 4A illustrates production of secreted Vstat120 by transient transfection in 293 cells. The cells (80% confluent) were left untreated (lane1) or transfected with either control pcDNA3.1lacZ vector (lane 2) or Vstat120 expression vector pcDNA3.1Vstat120- myc/his (Lane 3). Vstat120 produced by cells transfected with full length BAI1 expression vector was utilized as a size control (Lane 4).
Fig. 4B is a graph that illustrates a Transwell migration assay. Control or Vstat120 containing CM was tested for its ability to inhibit the migration of HDMECs and HUVECs in a Transwell migration assay.
Fig. 4C illustrates that CD36 function-blocking antibody prevents Vstat120 anti-angiogenic function. HDMECs were wounded, then either left untreated or treated with anti-CD36 function-blocking antibody at 10 µg/mL for 30 min. The cells were next treated with CM (as above) for 30 min, followed by treatment with 10% serum to induce cell migration. Final wound width was measured after 8 h and the distance migrated was calculated. Data is Mean +/- SEM. n=3 for each condition. * p<0.05 and ** not significant by Student's T test.
Fig. 4D is a graph that illustrates a scratch-wound migration assay. Confluent HDMECs were wounded, treated with CM and the cells allowed to migrate for 8 hrs, then fixed and stained with crystal violet. Fig. 4D also includes representative pictures of migrated cells. The black bars indicate initial wound width in micrometers. Distance of migration, percentage of wound closure, and speed of migration was quantified. The experiment was repeated twice with similar results. Data are expressed as mean +/- SEM; n=6 for each condition; * p<0.01 compared to Vstat120.

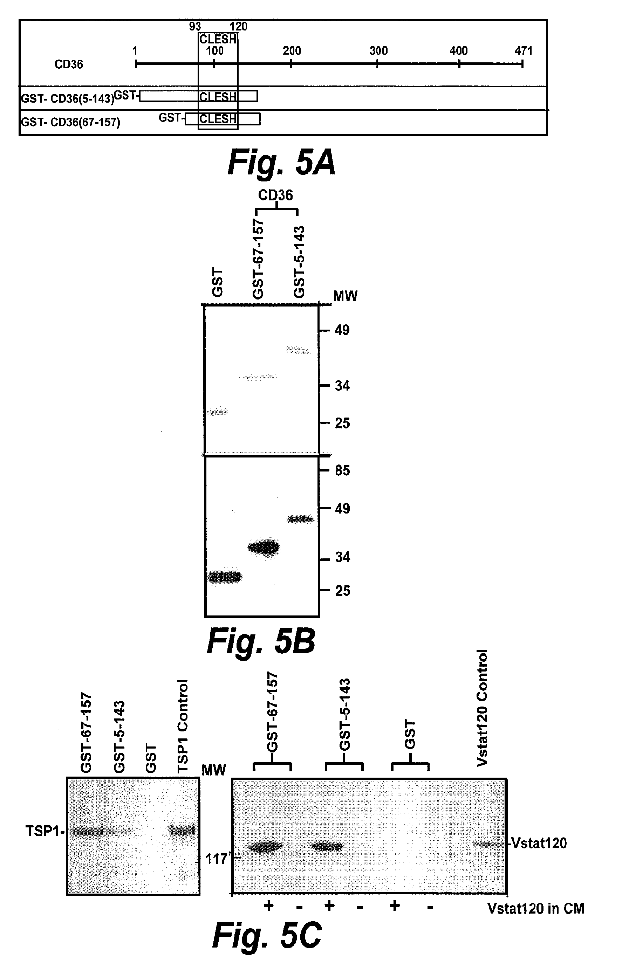

Figs. 5A-5C illustrate that Vstat120 binds to the purified CLESH domain of CD36.
Fig. 5A illustrates a schematic of CD36 structure with the CLESH domain. The two GST-CD36 constructs used (amino acids 5-143 and 67-157), both of which contain the CLESH domain (amino acids 93-120) are shown.
   The top panel of Fig. 5B illustrates a coomassie stained gel showing purified GST, and GST tagged recombinant proteins encoding for amino acids 67-157 and 5-143 of CD36. Proteins purified to near homogeneity and migrated at their predicted molecular weight. The bottom panel of Fig. 5B illustrates a Western blot analysis of each fusion protein probed with anti-GST monoclonal antibody (MAB3317 Chemicon International).
Fig. 5C illustrates a GST pull-down assay. GST alone or the two recombinant GST-CD36 peptides were bound to glutathione sepharose beads and CM from LN229 glioma cells stably expressing Vstat120 (+ lanes) or control cells (- lanes) were tested for protein interaction. A separate pull-down assay with CM from TSP1 expressing cells (LN229 clone C9) was used as a positive control. The bound proteins were eluted and analyzed for Vstat120 and TSP1 expression by western blot. Both the GST tagged CD36 containing recombinant peptides could pull down Vstat120 and TSP1 but not the purified GST. Positive control lanes are TCA precipitations of CM (collected serum-free after 96hrs) that express either Vstat120 or TSP1.

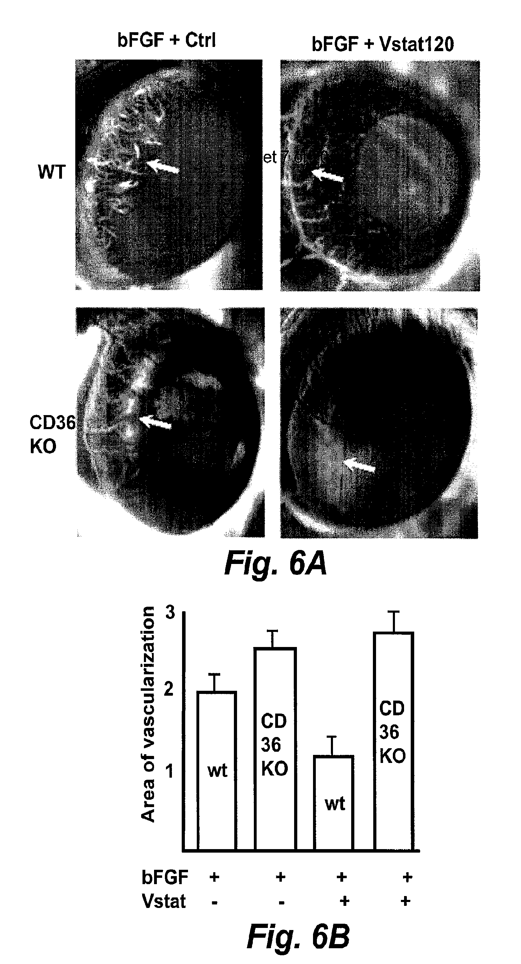

Fig. 6 illustrates that Vstat120 inhibits corneal angiogenesis in a CD36-dependent manner. Fig. 6A illustrates mice cornea at 5 days post implantation of pellets containing 25ng of bFGF and CM of 293 cells (50ng total CM protein) transfected with Vstat120 or vector control (Ctrl). Fig. 6A shows photographs show FITC-dextran labeled capillaries (arrow) progressing toward the pellet, previously inserted in the mouse cornea. Angiogenic response was quantified, as shown in the graph shown in Fig. 6B, by measuring the neovascular area in the cornea. Relative to the control (Fig. 6A, upper left), CM collected from Vstat120-expressing cells (Fig. 6A, upper right) impairs capillary formation by 40%. This effect is totally negated in CD36 knockout mice (Fig. 6A, bottom pictures). Each condition was carried out in at least 9 corneas. The values are expressed in means SE. Statistical analysis was performed using the ANOVA test, *p<0.05. 29

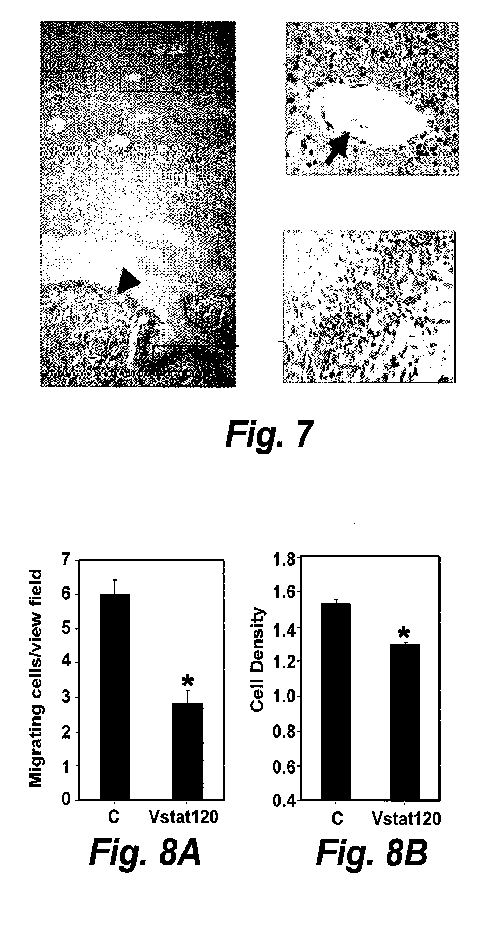

Figs. 7A and 7B illustrate that BAI1 expression is disrupted during tumorigenesis. Fig. 7A illustrates an autopsy specimen containing a glial blastoma and adjacent non-neoplastic white matter stained for BAI1 (left), and a higher magnification the adjacent brain (right, upper), and neoplastic tissue (right, lower) are also shown.
Figs. 8A and 8B illustrate that the BAI1 cleavage fragment of Vstat120 has anti-angiogenic properties. Fig. 8A shows the results from endothelial cell migration in a Boyden chamber assay. Fig. 8B shows endothelial cell proliferation in a crystal violet assay.

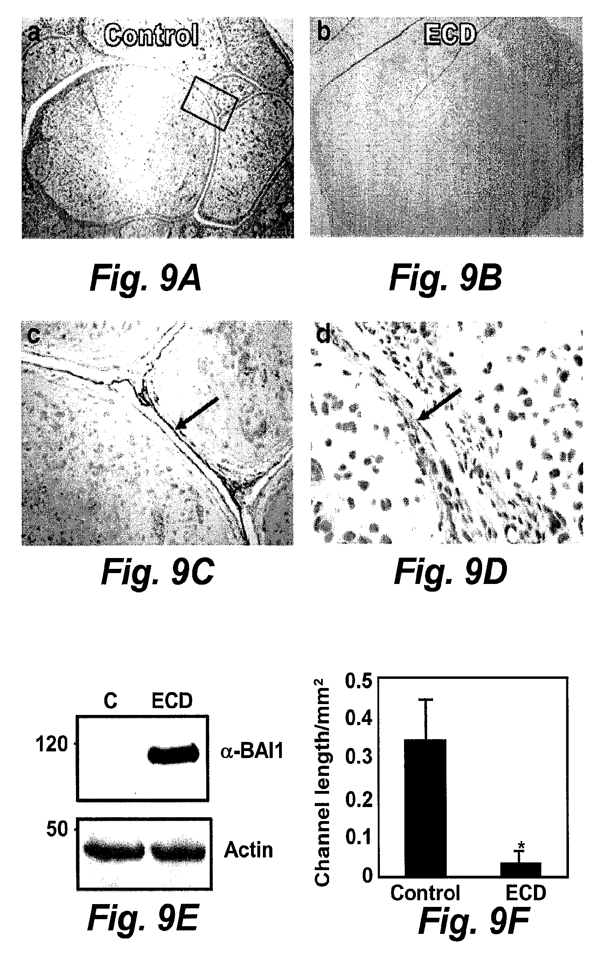

Figs. 9A-9F illustrate that the expression of Vstat120 inhibits angiogenesis *in vivo* in a matrigel plug assay. The length of vascular channels was measured after 14 days. Figs. 9A and 9B show representative H&E-stained sections of the control or Vstat120-expressing samples, respectively. Fig. 9C shows a higher magnification of the boxed region in Fig. 9A and illustrating vWF immunostaining of the endothelial cells lining the vascular channels (arrow). Fig. 9D shows that smooth muscle actin stained pericytes line the vascular channels (arrow). Fig. 9E shows a Western blot showing expression of Vstat120 in clones used in the matrigel experiment. C=vector control. Actin was the loading control. Fig. 9F shows a comparison of the average vascular channel length/surface area in plugs from control and Vstat120 expressing cells.

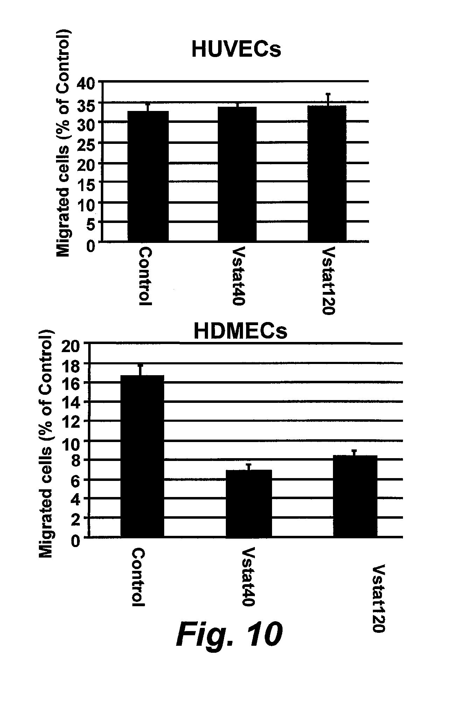

Fig. 10 illustrates that CD36 is required for the anti-angiogenesis function of Vstat40 and Vstat120 on HUVECs and HDMECs pretreated with conditioned media from 293 cells transfected with LacZ (Control), BAI1-S927A (Vstat40), or Vstat120 cDNA for 30 min. Media containing 10% serum was used as a chemoattractant and placed in the bottom chamber. After 8 h migrated cells were quantified.

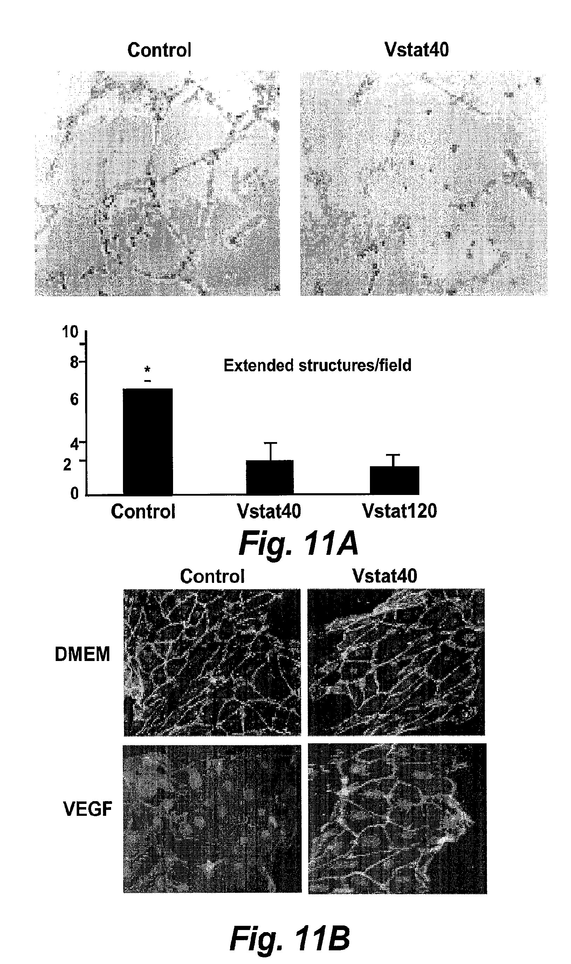

Fig. 11A illustrates that the BAI1 cleavage fragments Vstat40 and Vstat120 inhibit endothelial cord formation *in vitro.* HDMECs were grown on matrigel containing conditioned medium from 293 cells transfected with LacZ (Cont), BAI1-S927A (Vstat40), or Vstat120 cDNA. Enclosed structures (graph) were counted after 8 hr. Standard deviation is shown (n=4). * p<0.05 Student's T test.
Fig. 11B illustrates that Vstat40 and Vstat120 preserve endothelial adherens junctions. HDMECs were treated with conditioned medium from 293 cells transfected with LacZ (Cont), BAI1-S927A (Vstat40), or Vstat120 cDNA for 30 min. Cells were then left untreated or treated with VEGF (100 ng/ml) for 16h. Cells were fixed and immunostained using an anti-VE cadherin antibody (Ab) revealed by an FITC Ab. Note differences in "thickness" of cell membrane VE cadherin stain.

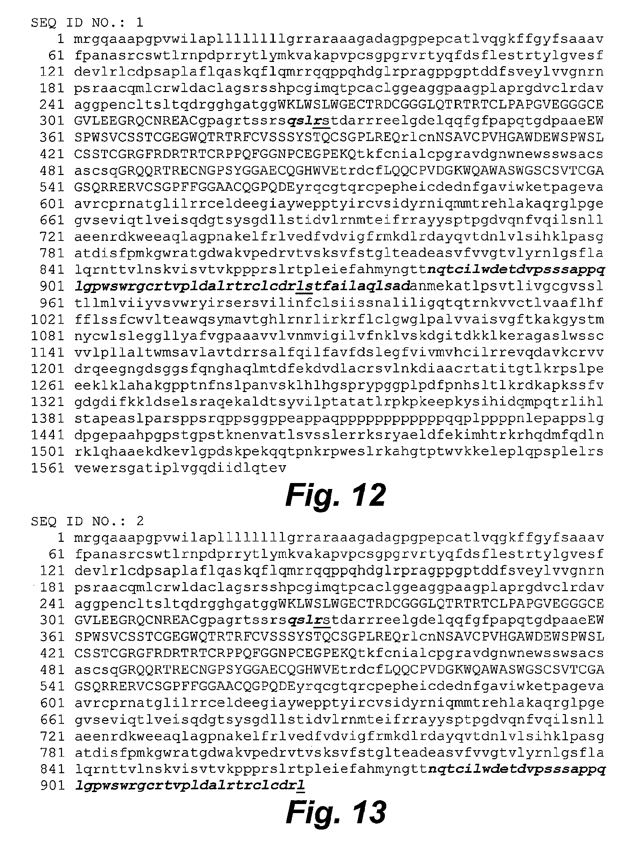

Fig. 12 illustrates the human BAI1 protein sequence SEQ ID NO.: 1. Five thrombospondin type I repeats are indicated -in large case letters. The sequence in bold represents the consensus GPS cleavage site used to generate Vstat120. The predicted cleavage site for the Vstat120 is in between the "Is" underlined sequence. The data indicates that the N-terminal cut that generates Vstat40 occurs in between the underlined "rs".
Fig. 13 illustrates a human Vstat120 protein sequence SEQ ID NO.: 2.

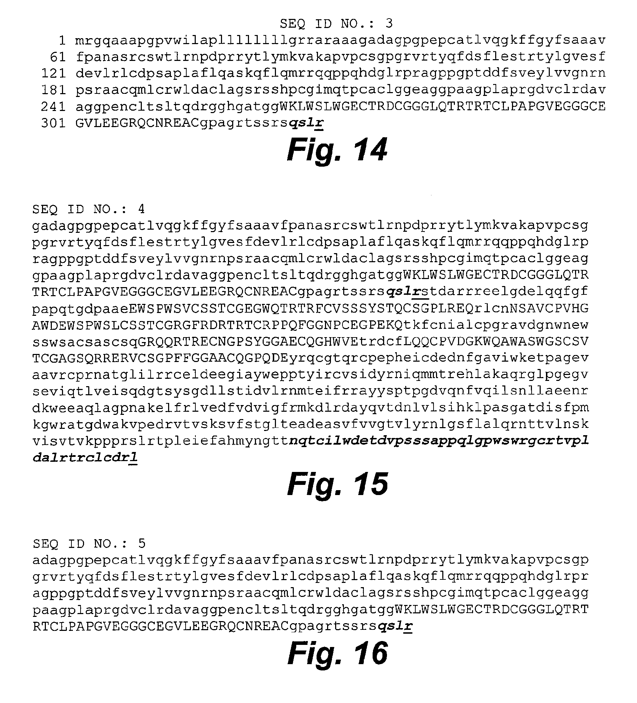

Fig. 14 illustrates a human Vstat40 protein sequence SEQ ID NO.: 3.
Fig. 15 illustrates a human Vstat120 protein sequence, not including the leader peptide sequence, (SEQ ID NO.: 4).
Fig. 16 illustrates a human Vstat40 protein sequence SEQ ID NO.: 5, not having a leader peptide sequence.

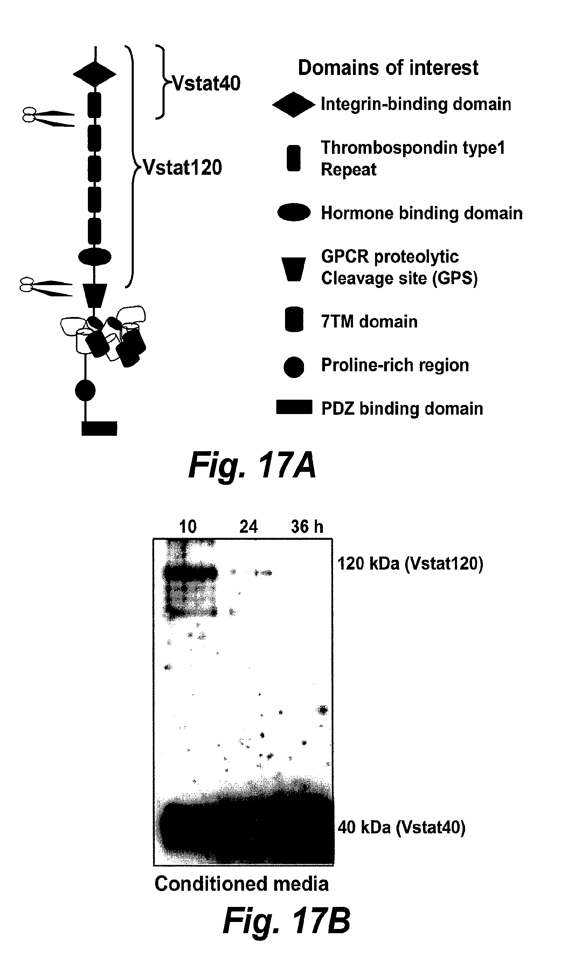

Fig. 17A illustrates schematically the structure of the 180 kDa BAI1 receptor.
Fig. 17B illustrates a Western blot showing that the extracellular domain of BAI1 is primarily processed into the secreted molecule Vstat40 in addition to Vstat120.

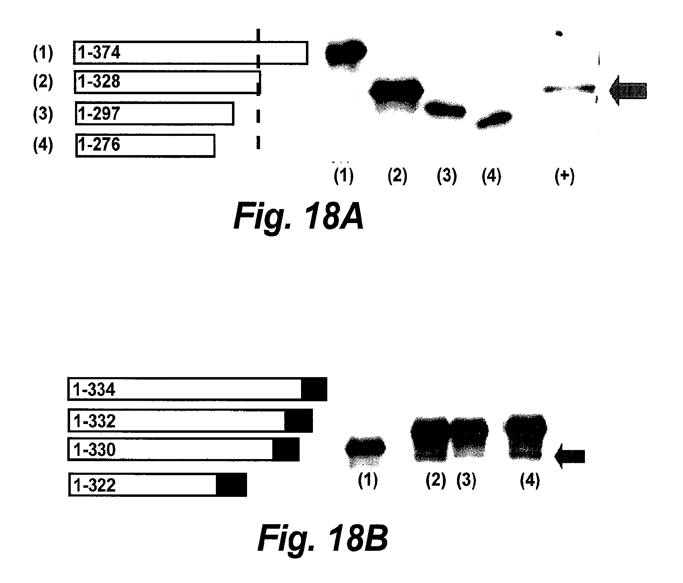

Fig. 18A illustrates that serial truncations of BAI1 N-terminal cDNA generate peptide products of corresponding size when transfected into LN229 glioma cells. Truncation at amino acid 328 generates a product of the approximate size of Vstat40 (arrow), indicating that the cleavage site is close to amino acid 328. Dashed vertical lines indicate the site of Vstat40 cleavage. Fragment 1-374 is still cleaved and generates a low amount of Vstat40,.
Fig. 18B illustrates the attachment of 3 kDa tags (dark shade) to constructs ending between amino acids 322 and 334. Constructs 2, 3, and 4 are still cleaved into Vstat40 while (1) is not, indicating that the cleavage site occurs between amino acids 322 and 330.

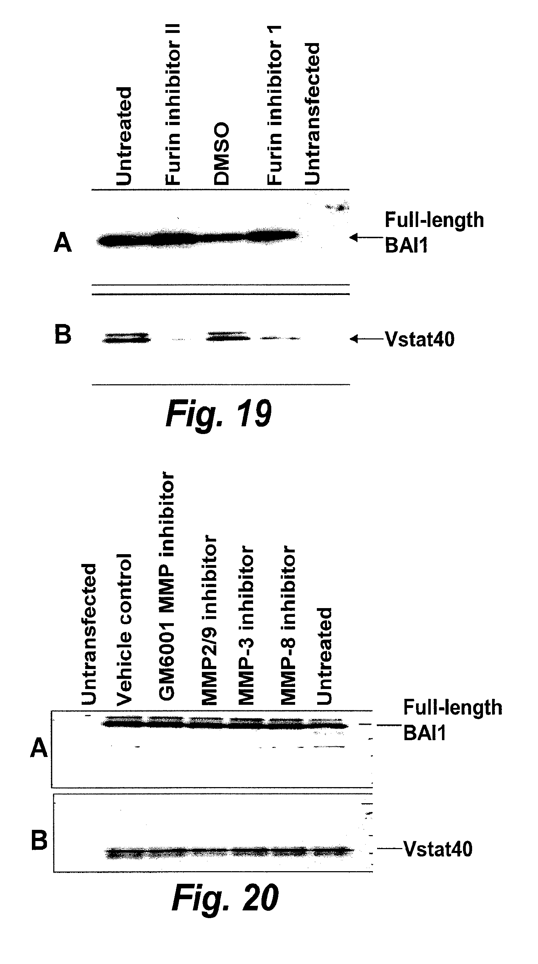

Fig. 19 illustrates that furin inhibitors abrogate Vstat40 processing. Fig. 19A shows full-length BAI1 protein in the whole cell lysate of transfected LN229 glioma cells. Fig. 19B shows that treatment of LN229 glioma cells transfected with BAI1 cDNA with two furin inhibitors abrogates processing and secretion of the Vstat40 fragment into conditioned media.
Fig. 20 illustrates that MMP inhibitors do not inhibit Vstat40 processing. (A) shows full-length BAI1 protein in the whole cell lysate of transfected LN229 glioma cells. (B) shows that treatment of LN229 glioma cells transfected with BAI1 cDNA with MMP inhibitors do not affect processing and secretion of the Vstat40 fragment into conditioned media.

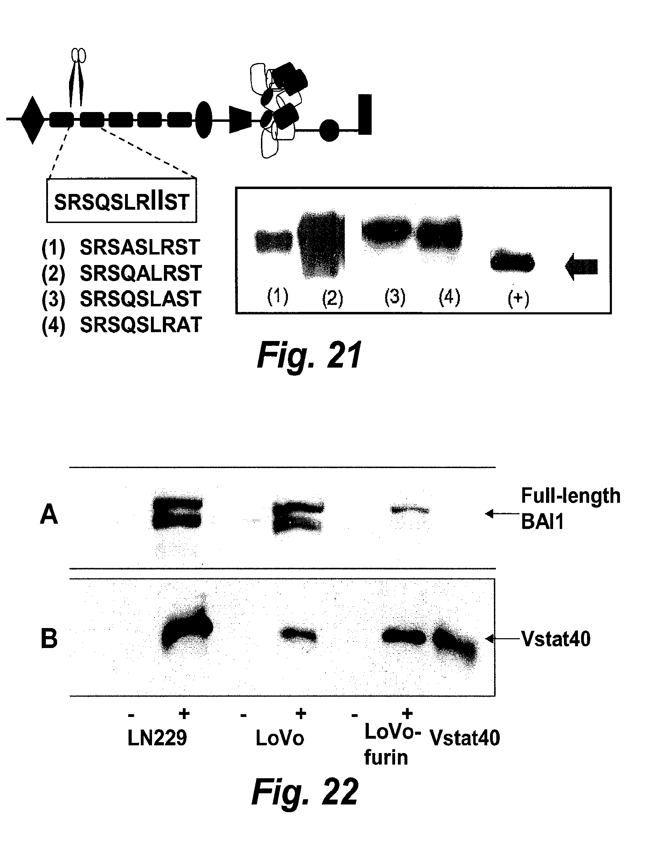

Fig. 21 illustrates that point mutations in the region of the Vstat40 processing site identify the key amino acids important for cleavage.
Fig. 22 illustrates that Vstat40 processing is abrogated in furin deficient LoVo adenocarcinoma cells. Fig. 22A shows full-length BAI1 protein in the whole cell lysate of transfected LoVo cells. Fig. 22B shows that Vstat40 processing is inhibited in furin-deficient LoVo human colon adenocarcinoma cell line and restored in LoVo cells stably transfected with furin cDNA.

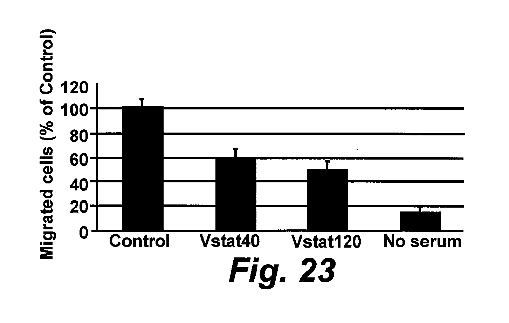

Fig. 23 illustrates that Vstat40 inhibits CD36+ (HDMEC) endothelial cell migration.

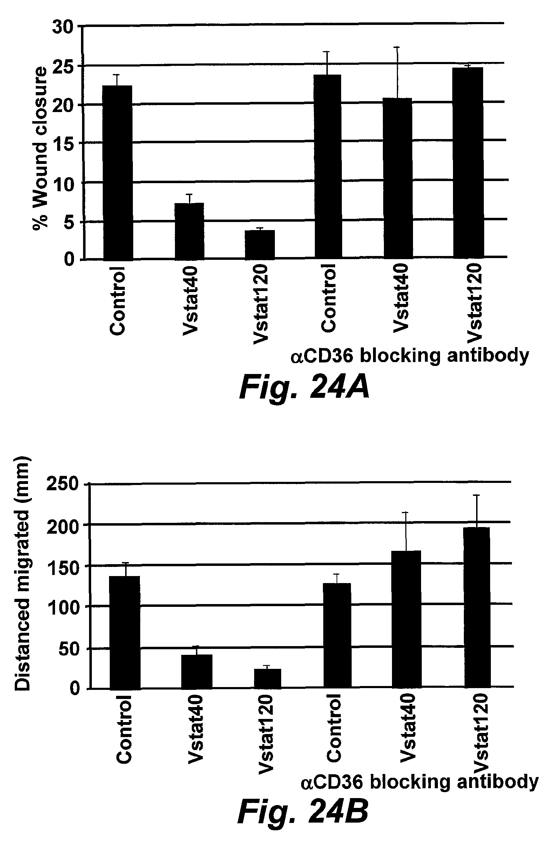

Figs. 24A and 24B illustrate that a CD36 blocking antibody inhibits Vstat40 effects on endothelial cell migration in a scratch wound assay. Fig. 24A shows the percent wound closure, and Fig. 24B shows distance migrated.

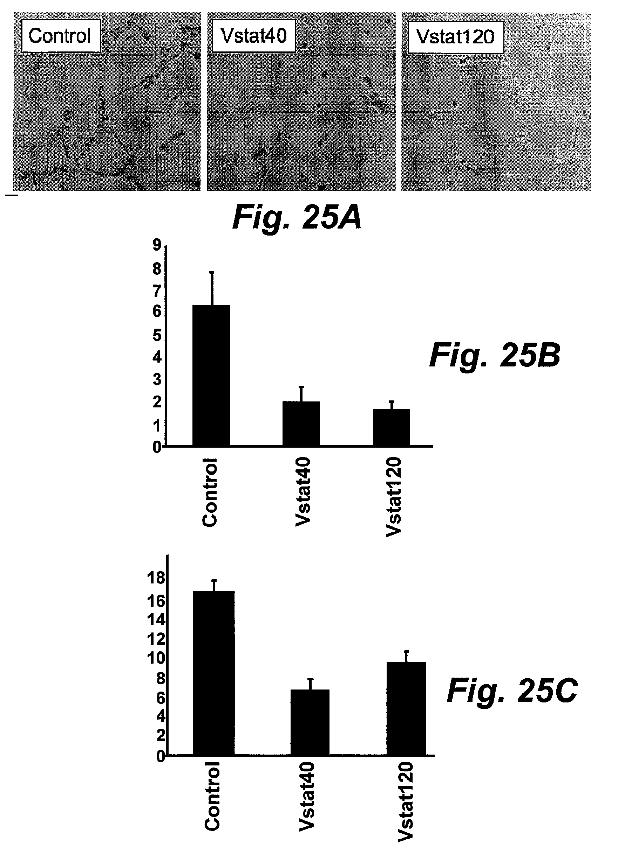

Fig 25A illustrates photomicrographs of endothelial cords cells cultured in medium without Vstat40 or Vstat120 (control), with Vstat40 or with Vstat120.
Fig. 25B illustrates a graph comparing the numbers of enclosed structures observed in the field of view of the cultures shown in Fig. 25A.
Fig. 25C illustrates a graph comparing the numbers of cords observed in the field of view of the cultures shown in Fig. 25A.

The details of some exemplary embodiments and aspects of the methods and systems of the present disclosure are set forth in the description below. Other features, objects, and advantages of the disclosure will be apparent to one of skill in the art upon examination of the following description, drawings, examples and claims. It is intended that all such additional systems, methods, features, and advantages be included within this description, be within the scope of the present disclosure, and be protected by the accompanying claims.

### DETAILED DESCRIPTION OF THE DISCLOSURE

Before the present disclosure is described in greater detail, it is to be understood that this disclosure is not limited to particular embodiments and aspects of the invention described, and as such may, of course, vary. It is also to be understood that the terminology used herein is for the purpose of describing particular embodiments and aspects of the invention only, and is not intended to be limiting, since the scope of the present disclosure will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the disclosure. The upper and lower limits of these smaller ranges may independently be included in the smaller ranges and are also encompassed within the disclosure, subject to any specifically excluded limit in the stated range. Where the stated range includes one or both of the limits, ranges excluding either or both of those included limits are also included in the disclosure.

Unless defined otherwise, all technical and scientific terms used herein have the same meaning as commonly understood by one of ordinary skill in the art to which this disclosure belongs. Although any methods and materials similar or equivalent to those described herein can also be used in the practice or testing of the present disclosure, the preferred methods and materials are now described.

The citation of any publication is for its disclosure prior to the filing date and should not be construed as an admission that the present disclosure is not entitled to antedate such publication by virtue of prior disclosure. Further, the dates of publication provided could be different from the actual publication dates that may need to be independently confirmed.

As will be apparent to those of skill in the art upon reading this disclosure, each of the individual embodiments and aspects of the invention described and illustrated herein has discrete components and features which may be readily separated from or combined with the features of any of the other several embodiments without departing from the scope of the present disclosure. Any recited method can be carried out in the order of events recited or in any other order that is logically possible.

Embodiments of the present disclosure will employ, unless otherwise indicated, techniques of medicine, organic chemistry, biochemistry, molecular biology, pharmacology, and the like, which are within the skill of the art. Such techniques are explained fully in the literature.

It must be noted that, as used in the specification and the appended claims, the singular forms "a," "an," and "the" include plural referents unless the context clearly dictates otherwise. Thus, for example, reference to "a support" includes a plurality of supports. In this specification and in the claims that follow, reference will be made to a number of terms that shall be defined to have the following meanings unless a contrary intention is apparent.

As used herein, the following terms have the meanings ascribed to them unless specified otherwise. In this disclosure, "comprises," "comprising," "containing" and "having" and the like can have the meaning ascribed to them in U.S. Patent law and can mean " includes," "including," and the like; "consisting essentially of" or "consists essentially" or the like, when applied to methods and compositions encompassed by the present disclosure refers to compositions like those disclosed herein, but which may contain additional structural groups, composition components or method steps (or analogs or derivatives thereof as discussed above). Such additional structural groups, composition components or method steps, etc., however, do not materially affect the basic and *novel* characteristic(s) of the compositions or methods, compared to those of the corresponding compositions or methods disclosed herein. "Consisting essentially of" or "consists essentially" or the like, when applied to methods and compositions encompassed by the present disclosure have the meaning ascribed in U.S. Patent law and the term is open-ended, allowing for the presence of more than that which is recited so long as basic or *novel* characteristics of that which is recited is not changed by the presence of more than that which is recited, but excludes prior art embodiments.

Prior to describing the various embodiments and aspects of the invention, the following definitions are provided and should be used unless otherwise indicated.

### Definitions

Generally the terms and phrases used herein have their art-recognized meaning which can be found by reference to standard texts, journal references and contexts known to those skilled in the art. The following definitions are provided to clarify their specific use in the context of this disclosure.

The term "expressed" or "expression" as used herein refers to the transcription from a gene to give an RNA nucleic acid molecule at least complementary in part to a region of one of the two nucleic acid strands of the gene. The term "expressed" or "expression" as used herein can also refer to the translation of RNA to produce a protein or peptide.

The term "fragment" as used herein can refer to, for example, an at least about 5, 10, 20, 30, 40, 50, 75, 100, 150, 200, 250, 300, 400, 500, 1000, or 2000, amino acid portion of an amino acid sequence, which portion is cleaved from a naturally occurring amino acid sequence by proteolytic cleavage by at least one protease, or is a portion of the naturally occurring amino acid sequence synthesized by chemical methods or using recombinant DNA technology (*e.g*., expressed from a portion of the nucleotide sequence encoding the naturally occurring amino acid sequence) known to one of skill in the art. "Fragment" may also refer to a portion, for example, of about 5%, about 10%, about 20%, about 30%, about 40%, about 50%, about 60%, about 70%, about 80%, about 90%, about 95%, or about 99% of a particular nucleotide sequence or amino acid sequence.

The term "gene expression controlling region" as used herein refers to nucleotide sequences that are associated with a coding sequence and which regulate, in whole or in part, expression of the coding sequence, for example, regulate, in whole or in part, the transcription of the coding sequence. The "gene expression controlling regions" may precede, but is not limited to preceding, the region of a nucleic acid sequence that is in the region 5' of the end of a coding sequence that may be transcribed into mRNA.

The terms "heterologous", "exogenous" and "foreign" are used interchangeably herein and in general refer to a biomolecule such as a nucleic acid or a protein that is not normally found in a certain organism or in a certain cell, tissue or other component contained in or produced by an organism.

The term "nucleic acid" as used herein refers to any linear or sequential array of nucleotides and nucleosides, for example cDNA, genomic DNA, mRNA, tRNA, siRNA, shRNA, miRNA, oligonucleotides, oligonucleosides and derivatives thereof. For ease of discussion, non-naturally occurring nucleic acids may be referred to herein as constructs. Nucleic acids can include bacterial plasmid vectors including expression, cloning, cosmid and transformation vectors such as, animal viral vectors such as, but not limited to, modified adenovirus, herpes virus, influenza virus, polio virus, pox virus, retroviruses such as avian leukosis virus (ALV) retroviral vector, a murine leukemia virus (MLV) retroviral vector, and a lentivirus vector, and the like and fragments thereof. In addition, the nucleic acid can be an LTR of an avian leukosis virus (ALV) retroviral vector, a murine leukemia virus (MLV) retroviral vector, or a lentivirus vector and fragments thereof. Nucleic acids can also include NL vectors such as NLB, NLD and NLA and fragments thereof and synthetic oligonucleotides such as chemically synthesized DNA or RNA. Nucleic acids can include modified or derivative nucleotides and nucleosides such as, but not limited to, halogenated nucleotides such as, but not only, 5-bromouracil, and derivative nucleotides such as biotin-labeled nucleotides.

The term "vector" and "nucleic acid vector" as used herein refers to a natural or synthetic single or double stranded plasmid or viral nucleic acid molecule that can be transfected or transformed into cells and replicate independently of, or within, the host cell genome. A circular double stranded vector can be linearized by treatment with an appropriate restriction enzyme based on the nucleotide sequence of the vector. A nucleic acid can be inserted into a vector by cutting the vector with restriction enzymes and ligating the desired pieces together.

The term "operably linked" refers to an arrangement of elements wherein the components so described are configured so as to perform their usual function. Gene expression controlling regions or promoters (*e.g.*, promoter components) operably linked to a coding sequence are capable of effecting the expression of the coding sequence. The controlling sequences need not be contiguous with the coding sequence, so long as they function to direct the expression thereof. Thus, for example, intervening untranslated yet transcribed sequences can be present between a promoter sequence and the coding sequence and the promoter sequence can still be considered "operably linked" to the coding sequence.

The terms "percent sequence identity", and "percent identity" as used in, for example, "% identical", "percent sequence homology", and "percent homology", as used in, for example, "% homology" and "percent sequence similarity", each refer to the degree of sequence matching between two nucleic acid sequences or two amino acid sequences as determined using the algorithm of Karlin & Attschul (1990) Proc. Natl. Acad. Sci. U.S.A. 87: 2264-2268, modified as in Karlin & Attschul (1993) Proc. Natl. Acad. Sci. U.S.A. 90: 5873-5877. Such an algorithm is incorporated into the NBLAST and XBLAST programs of Attschul et al. (1990) T. Mol. Biol. Q15: 403-410. BLAST nucleotide searches are performed with the NBLAST program. score = 100, word length = 12, to obtain nucleotide sequences homologous to a nucleic acid molecule of the disclosure. BLAST protein searches are performed with the XBLAST program, score=50, word length=3, to obtain amino acid sequences homologous to a reference amino acid sequence. To obtain gapped alignments for comparison purposes, Gapped BLAST is utilized as described in Attschul et al. (1997) Nucl. Acids Res. 25: 3389-3402. When utilizing BLAST and Gapped BLAST programs, the default parameters of the respective programs (e.g. XBLAST and NBLAST) are used. Other algorithms, programs and default settings may also be suitable such as, but not only, the GCG-Sequence Analysis Package of the U.K. Human Genome Mapping Project Resource Centre that includes programs for nucleotide or amino acid sequence comparisons.

The term "expression vector" as used herein refers to a nucleic acid vector that may further include at least one regulatory sequence operably linked to a nucleotide sequence coding for the desired polypeptide such as a variant Vstat polypeptide of the present disclosure. Regulatory sequences are well recognized in the art and may be selected to ensure good expression of the linked nucleotide sequence without undue experimentation by those skilled in the art. As used herein, the term "regulatory sequences" includes promoters, enhancers, and other elements that may control expression. Standard molecular biology textbooks such as Sambrook et al. eds "Molecular Cloning: A Laboratory Manual" 2nd ed. Cold Spring Harbor Press (1989) and Lodish et al., eds., "Molecular Cell Biology," Freeman (2000), may be consulted to design suitable expression vectors, promoters, and other expression control elements. It should be recognized, however, that the choice of a suitable expression vector depends upon multiple factors including the choice of the host cell to be transformed and/or the type of protein to be expressed.

Pharmaceutical compositions comprising the variant Vstat polypeptides of the present disclosure can be administered in dosages and by techniques well known to those skilled in the medical or veterinary arts, taking into consideration such factors as the age, sex, weight, species and condition of the particular patient, and the route of administration. The route of administration can be via any route that delivers a safe and effective dose of a composition of the present disclosure to the desired target such as a tumor, an eye and the like wherein angiogenesis inhibition is desirable. Pharmaceutical or therapeutic compositions can be administered alone, or can be co-administered or sequentially administered with other treatments or therapies. Forms of administration, including injectable administration, include, but are not limited to, intravenous, intraperitoneal, an intramuscular, an intrathecal, an intraarticular, an intrapulmonary, an intraperitoneal, a retroperitoneal, an intrapleural, a subcutaneous, a percutaneous, a transmucosal, an intranasal, an oral, a gastro-intestinal, and an intraocular route of administration of such as sterile solutions, suspensions or emulsions. A particularly advantageous route of delivery of the compositions of the disclosure to a tumor and the like is to directly introduce the composition into a blood vessel leading into the treatable area.

Pharmaceutical compositions may be administered in admixture with a suitable carrier, diluent, or excipient such as sterile water, physiological saline, glucose, or the like. The compositions can contain auxiliary substances such as wetting or emulsifying agents, pH buffering agents, adjuvants, gelling or viscosity enhancing additives, preservatives, flavoring agents, colors, and the like, depending upon the route of administration and the preparation desired. Standard pharmaceutical texts, such as "Remmington's Pharmaceutical Science," 17th edition, 1985 may be consulted to prepare suitable preparations, without undue experimentation. The effective dosage and route of administration are determined by the therapeutic range and nature of the compound, and by known factors, such as the age, weight, and condition of the host, as well as LD₅₀ and other screening procedures that are known and do not require undue experimentation. Dosages can generally range from a few hundred micrograms to a few grams administered as a bolus or over a sustained period as determined by the medical condition and need of a subject animal or human. The term "sustained" as used herein refers to any extended period ranging from several minutes to years.

The term "pharmaceutically acceptable" as used herein refers to a compound or combination of compounds that while biologically active will not damage the physiology of the recipient human or animal to the extent that the viability of the recipient is comprised. Preferably, the administered compound or combination of compounds will elicit, at most, a temporary detrimental effect on the health of the recipient human or animal is reduced.

The term "intravascularly" as used herein refers to a route of delivering a fluid, such as a pharmaceutically acceptable composition, to a blood vessel.

The term "dosage" as used herein refers to the amount of a Vstat polypeptide of the present disclosure administered to an animal or human. Suitable dosage units for use in the methods of the present disclosure range from mg/kg body weight of the recipient subject to mg/kg. The therapeutic agent may be delivered to the recipient as a bolus or by a sustained (continuous or intermittent) delivery. Delivery of a dosage may be sustained over a period, which may be in the order of a few minutes to several days, weeks or months, or may be administer chronically for a period of years.

In this regard, during the period of administration, each individual patient should be examined to see how they are reacting to the treatment of the present disclosure. For instance, the patient should be examined for the above noted possible adverse reactions. The disease tissue, *e.g.*, tumor, should also be examined, *e.g.*, by biopsy or soft X-ray microscopy, to see whether the period of administration and/or dose should be modified.

In view of the above, the period of administration may be, but is not limited to, from about 1 day to about 1 week, about 1 week to 6 months, about 1 week to 3 months, about 2 weeks to 1 month, and about 2 to 3 weeks. If the period of administration is too long, the period of recovery between periods of administration is increased and adverse impacts on the patient's health are more likely. If the period of administration is too short, the disease tissue, *e.g*., tumor, may not be reduced.

The term "directly delivering" as used herein refers to delivering a pharmaceutical preparation into a mass of target cells or population of cells within a defined location within a subject human or animal, whereby the preparation is not delivered by administration into the circulatory system to be distributed throughout the body rather than specificially or mainly to the target tissue. It is expected that the administration may be by injection near the disease tissue, *e.g*., tumor mass, to minimize side effects, although another advantageous route is expected to be intravascularly, and most advantageously into a vessel leading into the area to be treated. The manner of administration may also be transdermal, intramuscular, topical, subcutaneous, intracavity, peristaltic. Regarding injection near the disease tissue, *e.g*., tumor mass, it is expected that micro-pumps may be implanted in or near the disease tissue, *e.g*., tumor mass, to administer the dose in a manner similar to insulin pumps.

The compositions of the present disclosure may comprise a pharmaceutical composition comprising a polypeptide(s) of the present disclosure and at least one pharmaceutically acceptable carrier or excipient. As used herein, the terms "pharmaceutically acceptable", "physiologically tolerable" and grammatical variations thereof, as they refer to compositions, carriers, diluents and reagents, are used interchangeably and represent that the materials are capable of administration to or upon a mammal without the production of undesirable physiological effects such as nausea, dizziness, gastric upset and the like.

The preparation of a pharmacological composition that contains active ingredients dissolved or dispersed therein is well understood in the art and need not be limited based on formulation. Typically such compositions are prepared as injectables either as liquid solutions or suspensions, however, solid forms suitable for solution, or suspensions, in liquid prior to use can also be prepared. The preparation can also be emulsified.

The active ingredient can be mixed with excipients which are pharmaceutically acceptable and compatible with the active ingredient and in amounts suitable for use in the therapeutic methods described herein. Suitable excipients are, for example, water, saline, dextrose, glycerol, ethanol or the like and combinations thereof. In addition, if desired, the composition can contain minor amounts of auxiliary substances such as wetting or emulsifying agents, pH buffering agents and the like which enhance the effectiveness of the active ingredient.

Physiologically tolerable carriers are well known in the art. Exemplary of liquid carriers are sterile aqueous solutions that contain no materials in addition to the active ingredients and water, or contain a buffer such as sodium phosphate at physiological pH value, physiological saline or both, such as phosphate-buffered saline. Still further, aqueous carriers can contain more than one buffer salt, as well as salts such as sodium and potassium chlorides, dextrose, polyethylene glycol and other solutes.

Liquid compositions can also contain liquid phases in addition to and to the exclusion of water. Exemplary of such additional liquid phases are glycerin, vegetable oils such as cottonseed oil, and water-oil emulsions.

A therapeutic composition contains an angiogenesis-inhibiting amount of an active compound of the present disclosure, typically formulated to contain an amount of at least about 0.1 weight percent of active compound of the present disclosure per weight of total therapeutic composition. A weight percent is a ratio by weight of active compound to total composition. Thus, for example, about 0.1 weight percent is about 0.1 grams of active compound per 100 grams of total composition.

While not wishing to be bound by theory, the dosage is expected to depend upon factors such as period of administration, stage of disease tissue, *e.g*., tumor, endogenous factors, disease tissue, *e.g*., tumor, behavior, and the patient's individual physiology. For shorter periods of administration, higher dosages are generally used. For later stage disease tissue, *e.g*., tumors, the dosage should generally be higher. For example, if the tumor has metastasized, the dosage should generally be higher. Dosages will generally be higher for more resistant and/or aggressive disease tissue, *e.g*., tumors. The dosage should also be affected by the patient's individual physiology. For instance, if the individual is healthy, the dosage can be higher. Also, if the individual is tolerant to the composition of the present disclosure, the dosage should generally be higher. Conversely, if an individual has adverse reactions, the treatment method of the present disclosure may not be appropriate or the dosage should generally be reduced.

In this regard, during the initial period of administration, the dosage should generally be low and then can be gradually increased depending upon how the patient reacts to the treatment of the present disclosure. For instance, during the first week of administration the dosage should generally be small. After the first week, if there are no adverse reactions, the dosage may be increased during the second week. After the second week, if there are still no adverse reactions, the dosage may be increased even further during the third week.

In view of the above and in view of the data shown in the examples of the present application, after the initial period of administration, the patient should be allowed to recover during which time the composition of the present disclosure is not administered. The period of recovery between periods of administration is expected to depend upon factors such as the health of the patient. If the patient is generally healthy, the period of recovery between periods of administration may be less.

The compounds of the present disclosure may also be administered in combination with other angiogenesis inhibitors. For instance, if the compounds of the present disclosure and the other angiogenesis inhibitors have different targets, the effect is expected to be at least additive and side effects would be expected to decrease. In particular, the different targets may be different mechanisms and/or different cells. In this regard, the compounds of the present disclosure may target both disease tissue, *e.g*., tumor cells, and capillary endothelial cells. Accordingly, it is expected that the compounds of the present disclosure may be used with compositions which target endothelial cells or disease tissue cells.

The term "polypeptides" includes proteins and fragments thereof. Polypeptides are disclosed herein as amino acid residue sequences. Those sequences are written left to right in the direction from the amino to the carboxy terminus. In accordance with standard nomenclature, amino acid residue sequences are denominated by either a three letter or a single letter code as indicated as follows: Alanine (Ala, A), Arginine (Arg, R), Asparagine (Asn, N), Aspartic Acid (Asp, D), Cysteine (Cys, C), Glutamine (Gin, Q), Glutamic Acid (Glu, E), Glycine (Gly, G), Histidine (His, H), Isoleucine (Ile, I), Leucine (Leu, L), Lysine (Lys, K), Methionine (Met, M), Phenylalanine (Phe, F), Proline (Pro, P), Serine (Ser, S), Threonine (Thr, T), Tryptophan (Trp, W), Tyrosine (Tyr, Y), and Valine (Val, V).

"Variant" refers to a polypeptide or polynucleotide that differs from a reference polypeptide or polynucleotide, but retains essential properties. A typical variant of a polypeptide differs in amino acid sequence from another, reference polypeptide. Generally, differences are limited so that the sequences of the reference polypeptide and the variant are closely similar overall and, in many regions, identical. A variant and reference polypeptide may differ in amino acid sequence by one or more modifications (*e.g*., substitutions, additions, and/or deletions). A variant of a polypeptide includes conservatively modified variants. A substituted or inserted amino acid residue may or may not be one encoded by the genetic code. A variant of a polypeptide may be naturally occurring, such as an allelic variant, or it may be a variant that is not known to occur naturally.

Modifications and changes can be made in the structure of the polypeptides of this disclosure and still obtain a molecule having similar characteristics as the polypeptide (*e.g*., a conservative amino acid substitution). For example, certain amino acids can be substituted for other amino acids in a sequence without appreciable loss of activity. Because it is the interactive capacity and nature of a polypeptide that defines that polypeptide's biological functional activity, certain amino acid sequence substitutions can be made in a polypeptide sequence and nevertheless obtain a polypeptide with like properties.

The term "conservative substitutions" as used herein refers to modifications of a polypeptide that involve the substitution of one or more amino acids for amino acids having similar biochemical properties that do not result in change or loss of a biological or biochemical function of the polypeptide. These "conservative substitutions" are likely to have minimal impact on the activity of the resultant protein. Amino acids that may be substituted for an original amino acid in a protein, and which are generally regarded as conservative substitutions are (original residue: conservative substitution): Ala: ser; Arg: lys; Asn: gin, his; Asp: glu; Cys: ser; Gin: asn; Glu: asp; Gly: pro; His: asn, gin; Ile: leu, val; Leu: ile, val; Lys: arg, gin; Met: leu, ile; Phe: met, leu, tyr; Ser: thr; Thr: ser; Trp: tyr; Tyr: trp, phe; Val: ile, leu. One or more conservative changes, or up to ten conservative changes, can be made in a polypeptide without changing a biochemical function of the polypeptide. For example, one or more conservative changes can be made in a Vstat40 or Vstat120 polypeptide without changing its ability to bind to CD36.

In making such changes, the hydropathic index of amino acids can be considered. The importance of the hydropathic amino acid index in conferring interactive biologic function on a polypeptide is generally understood in the art. It is known that certain amino acids can be substituted for other amino acids having a similar hydropathic index or score and still result in a polypeptide with similar biological activity. Each amino acid has been assigned a hydropathic index on the basis of its hydrophobicity and charge characteristics. Those indices are: isoleucine (+4.5); valine (+4.2); leucine (+3.8); phenylalanine (+2.8); cysteine/cysteine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5).

It is believed that the relative hydropathic character of the amino acid determines the secondary structure of the resultant polypeptide, which in turn defines the interaction of the polypeptide with other molecules, such as enzymes, substrates, receptors, antibodies, antigens, and the like. It is known in the art that an amino acid can be substituted by another amino acid having a similar hydropathic index and still obtain a functionally equivalent polypeptide. In such changes, the substitution of amino acids whose hydropathic indices are within ± 2 is preferred, those within ± 1 are particularly preferred, and those within ± 0.5 are even more particularly preferred.

Substitution of like amino acids can also be made on the basis of hydrophilicity, particularly, where the biological functional equivalent polypeptide or peptide thereby created is intended for use in immunological embodiments. The following hydrophilicity values have been assigned to amino acid residues: arginine (+3.0); lysine (+3.0); aspartate (+3.0 ± 1); glutamate (+3.0 ± 1); serine (+0.3); asparagine (+0.2); glutamnine (+0.2); glycine (0); proline (-0.5 ± 1); threonine (-0.4); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4). It is understood that an amino acid can be substituted for another having a similar hydrophilicity value and still obtain a biologically equivalent, and in particular, an immunologically equivalent polypeptide. In such changes, the substitution of amino acids whose hydrophilicity values are within ± 2 is preferred, those within ± 1 are particularly preferred, and those within ± 0.5 are even more particularly preferred.

As outlined above, amino acid substitutions are generally based on the relative similarity of the amino acid side-chain substituents, for example, their hydrophobicity, hydrophilicity, charge, size, and the like. Exemplary substitutions that take various of the foregoing characteristics into consideration are well known to those of skill in the art and include (original residue: exemplary substitution): (Ala: Gly, Ser), (Arg: Lys), (Asn: Gin, His), (Asp: Glu, Cys, Ser), (Gin: Asn), (Glu: Asp), (Gly: Ala), (His: Asn, Gin), (Ile: Leu, Val), (Leu: Ile, Val), (Lys: Arg), (Met: Leu, Tyr), (Ser: Thr), (Thr: Ser), (Tip: Tyr), (Tyr: Trp, Phe), and (Val: Ile, Leu). Embodiments of this disclosure thus contemplate functional or biological equivalents of a polypeptide as set forth above. In particular, embodiments of the polypeptides can include variants having about 50%, 60%, 70%, 80%, 90%, and 95% sequence identity to the polypeptide of interest.

"Identity," as known in the art, is a relationship between two or more polypeptide sequences, as determined by comparing the sequences. In the art, "identity" also means the degree of sequence relatedness between polypeptides as determined by the match between strings of such sequences. "Identity" and "similarity" can be readily calculated by known methods, including, but not limited to, those described in (Computational Molecular Biology, Lesk, A. M., Ed., Oxford University Press, New York, 1988; Biocomputing: Informatics and Genome Projects, Smith, D. W., Ed., Academic Press, New York, 1993; Computer Analysis of Sequence Data, Part I, Griffin, A. M., and Griffin, H. G., Eds., Humana Press, New Jersey, 1994; Sequence Analysis in Molecular Biology, von Heinje, G., Academic Press, 1987; and Sequence Analysis Primer, Gribskov, M. and Devereux, J., Eds., M Stockton Press, New York, 1991; and Carillo, H., and Lipman, D., SIAM J Applied Math., 48: 1073 (1988).

Preferred methods to determine identity are designed to give the largest match between the sequences tested. Methods to determine identity and similarity are codified in publicly available computer programs. The percent identity between two sequences can be determined by using analysis software (*e.g*., Sequence Analysis Software Package of the Genetics Computer Group, Madison Wis.) that incorporates the Needelman and Wunsch, (J. Mol. Biol., 48: 443-453, 1970) algorithm (*e.g*., NBLAST, and XBLAST). The default parameters are used to determine the identity for the polypeptides of the present disclosure.

By way of example, a polypeptide sequence may be identical to the reference sequence, that is 100% identical, or it may include up to a certain integer number of amino acid alterations as compared to the reference sequence such that the % identity is less than 100%. Such alterations are selected from: at least one amino acid deletion, substitution, including conservative and non-conservative substitution, or insertion, and wherein said alterations may occur at the amino- or carboxy-terminal positions of the reference polypeptide sequence or anywhere between those terminal positions, interspersed either individually among the amino acids in the reference sequence or in one or more contiguous groups within the reference sequence. The number of amino acid alterations for a given % identity is determined by multiplying the total number of amino acids in the reference polypeptide by the numerical percent of the respective percent identity (divided by 100) and then subtracting that product from said total number of amino acids in the reference polypeptide.

Conservative amino acid variants can also comprise non-naturally occurring amino acid residues. Non-naturally occurring amino acids include, without limitation, trans-3-methylproline, 2,4-methanoproline, cis-4-hydroxyproline, trans-4-hydroxyproline, N-methylglycine, allo-threonine, methylthreonine, hydroxy-ethylcysteine, hydroxyethylhomocysteine, nitro-glutamine, homoglutamine, pipecolic acid, thiazolidine carboxylic acid, dehydroproline, 3- and 4-methylproline, 3,3-dimethylproline, tert-leucine, norvaline, 2-azaphenyl-alanine, 3-azaphenylalanine, 4-azaphenylalanine, and 4-fluorophenylalanine. Several methods are known in the art for incorporating non-naturally occurring amino acid residues into proteins. For example, an *in vitro* system can be employed wherein nonsense mutations are suppressed using chemically aminoacylated suppressor tRNAs. Methods are known in the art for synthesizing amino acids and aminoacylating tRNA. Transcription and translation of plasmids containing nonsense mutations is carried out in a cell-free system comprising an *E. coli* S30 extract and commercially available enzymes and other reagents. Proteins are purified by chromatography. (Robertson, et al., J. Am. Chem. Soc., 113: 2722, 1991; Ellman, et al., Methods Enzymol., 202: 301, 1991; Chung, et al., Science, 259: 806-9, 1993; and Chung, et al., Proc. Natl. Acad. Sci. U S A , 90: 10145-9, 1993). In a second method, translation is carried out in Xenopus oocytes by microinjection of mutated mRNA and chemically aminoacylated suppressor tRNAs (Turcatti, et al., J. Biol. Chem., 271: 19991-8, 1996). Within a third method, *E. coli* cells are cultured in the absence of a natural amino acid that is to be replaced (*e.g*., phenylalanine) and in the presence of the desired non-naturally occurring amino acid(s) (*e.g*., 2-azaphenylalanine, 3-azaphenylalanine, 4-azaphenylalanine, or 4-fluorophenylalanine). The non-naturally occurring amino acid is incorporated into the protein in place of its natural counterpart. (Koide, et al., Biochem., 33: 7470-6, 1994). Naturally occurring amino acid residues can be converted to non-naturally occurring species by *in vitro* chemical modification. Chemical modification can be combined with site-directed mutagenesis to further expand the range of substitutions (Wynn, et al., Protein Sci., 2: 395-403, 1993).

As used herein, the term "nucleic acid molecule" is intended to include DNA molecules (*e.g.*, cDNA or genomic DNA), RNA molecules (*e.g*., mRNA), analogs of the DNA or RNA generated using nucleotide analogs, and derivatives, fragments and homologs thereof. The nucleic acid molecule can be single-stranded or double-stranded, but advantageously is double-stranded DNA. An "isolated" nucleic acid molecule is one that is separated from other nucleic acid molecules that are present in the natural source of the nucleic acid. A "nucleoside" refers to a base linked to a sugar. The base may be adenine (A), guanine (G) (or its substitute, inosine (I)), cytosine (C), or thymine (T) (or its substitute, uracil (U)). The sugar may be ribose (the sugar of a natural nucleotide in RNA) or 2-deoxyribose (the sugar of a natural nucleotide in DNA). A "nucleotide" refers to a nucleoside linked to a single phosphate group.

As used herein, the term "oligonucleotide" refers to a series of linked nucleotide residues, which oligonucleotide has a sufficient number of nucleotide bases to be used in a PCR reaction. A short oligonucleotide sequence may be based on, or designed from, a genomic or cDNA sequence and is used to amplify, confirm, or reveal the presence of an identical, similar or complementary DNA or RNA in a particular cell or tissue. Oligonucleotides may be chemically synthesized and may be used as primers or probes. Oligonucleotide means any nucleotide of more than 3 bases in length used to facilitate detection or identification of a target nucleic acid, including probes and primers.

The term "angiogenesis" as used herein is the process of new blood vessel growth from existing blood vessels. It is used to refer to growth under both normal physiological conditions and those during diseased states such as, but not limited to, tumor growth.

The term "cancer", as used herein shall be given its ordinary meaning and is a general term for diseases in which abnormal cells divide without control. Cancer cells can invade nearby tissues and can spread through the bloodstream and lymphatic system to other parts of the body. A "tumor" refers to solid tumors beyond 1-2 mm in size resulting from the abnormal growth of tissue, and involving the formation of new blood vessels.

When normal cells lose their ability to behave as a specified, controlled and coordinated unit, a tumor is formed. Generally, a solid tumor is an abnormal mass of tissue that usually does not contain cysts or liquid areas (some brain tumors do have cysts and central necrotic areas filled with liquid). A single tumor may even have different populations of cells within it with differing processes that have gone awry. Solid tumors may be benign (not cancerous), or malignant (cancerous). Different types of solid tumors are named for the type of cells that form them. Examples of solid tumors are sarcomas, carcinomas, and lymphomas. Leukemias (cancers of the blood) generally do not form solid tumors.

There are several main types of cancer, for example, carcinoma is cancer that begins in the skin or in tissues that line or cover internal organs (epithelium). Sarcoma is cancer that begins in bone, cartilage, fat, muscle, blood vessels, or other connective or supportive tissue. Leukemia is cancer that starts in blood-forming tissue such as the bone marrow, and causes large numbers of abnormal blood cells to be produced and enter the bloodstream. Lymphoma is cancer that begins in the cells of the immune system. Representative cancers include, but are not limited to, bladder cancer, breast cancer, colorectal cancer, endometrial cancer, head & neck cancer, leukemia, lung cancer, lymphoma, melanoma, non-small-cell lung cancer, ovarian cancer, prostate cancer, testicular cancer, uterine cancer, cervical cancer. This includes the gliomas described within as well as types derived from other tissues such as but not limited to carcinomas, lymphomas, leukemias and sarcomas.

The term "angiogenesis-stimulating growth factor" refers to those compounds capable of stimulating the growth of new blood vessels either *in vitro*, *in vivo*, or both. These factors are also referred to herein as "pro-angiogenic".

The term "angiogenesis inhibitor" refers to those compounds capable of inhibiting the growth of new blood vessels either in vitro, *in vivo,* or both. These factors are also referred to herein as "anti-angiogenic".

The term "metastasis" refers to the ability of cancer cells to break away from a primary tumor, penetrate into lymphatic or blood vessels, circulate through the bloodstream, and grow in a distant focus in normal tissues or organs elsewhere in the body.

The term "homologue" refers to proteins or peptides structurally similar to BAI1 or the protein fragments similar to Vstat120 or Vstat40 resulting from proteins or peptides similar to BAI1.

### Angiogenesis

Angiogenesis is the formation and growth of new blood vessels from pre-existing vessels and is an important natural process occurring in the body, both in healthy and in disease states. Angiogenesis occurs in the healthy body during wound healing, restoring blood flow to tissues after injury or insult. It also occurs normally in females, during the monthly reproductive cycle serving to rebuild the uterine lining as well as during pregnancy, to build the placenta, for circulation between mother and fetus.

In the healthy individual, the body controls angiogenesis through a series of "on" and "off" switches, known as angiogenesis-stimulating growth factors (pro-angiogenic) including, Angiogenin, Angiopoietin-1, Del-1, Fibroblast growth factors: acidic (aFGF) and basic (bFGF), Follistatin, Granulocyte colony-stimulating factor (G-CSF), Hepatocyte growth factor (HGF) /scatter factor (SF), Interleukin-8 (IL-8), Leptin, Midkine, Placental growth factor, Platelet-derived endothelial cell growth factor (PD-ECGF), Platelet-derived growth factor-BB (PDGF-BB), Pleiotrophin (PTN), Progranulin, Proliferin, Transforming growth factor-alpha (TGF-alpha), Transforming growth factor-beta (TGF-beta), Tumor necrosis factor-alpha (TNF-alpha), Vascular endothelial growth factor (VEGF)/vascular permeability factor (VPF). Typical known angiogenesis inhibitors include Angioarrestin, Angiostatin (plasminogen fragment), Anti-angiogenic antithrombin III, BAI1, BAI2, BAI3, Cartilage-derived inhibitor (CDI), CD59 complement fragment, Endostatin (collagen XVIII fragment), Fibronectin fragment, Gro-beta, Heparinases, Heparin hexasaccharide fragment, Human chorionic gonadotropin (hCG), Interferon alpha/beta/gamma, Interferon inducible protein (IP-10), Interleukin-12, Kringle 5 (plasminogen fragment), Metalloproteinase inhibitors (TIMPs), 2-Methoxyestradiol, Placental ribonuclease inhibitor, Plasminogen activator inhibitor, Platelet factor-4 (PF4), Prolactin 16kD fragment, Proliferin-related protein (PRP), Retinoids, Tetrahydrocortisol-S, Thrombospondin-1 (TSP-1), Transforming growth factor-beta (TGF-b), Vasculostatin, Vasostatin (calreticulin fragment).

When angiogenic growth factors are produced in excess of angiogenesis inhibitors, the balance of these is tipped in favor of blood vessel growth, while when inhibitors are present in excess of stimulators, angiogenesis is stopped. In the normal, healthy body a perfect balance of angiogenesis modulators is maintained.

In many diseases states, the body loses control over angiogenesis. Angiogenesis-dependent diseases result when new blood vessels either grow excessively or insufficiently. Diseases involving excessive blood vessel growth include but are not limited to, cancers, age-related macular degeneration, chronic inflammatory disease, rheumatoid arthritis, and psoriasis all occur when diseased cells produce abnormal amounts of pro-angiogenic factors, overwhelming the effects of natural angiogenesis inhibitors. In these conditions, new blood vessels feed the diseased tissues, destroy normal tissues, and in the case of cancer, the new vessels allow tumor cells to escape into the circulation and lodge in other organs (tumor metastasis). Insufficient angiogenesis plays a role in conditions such as coronary artery disease, stroke, and delayed wound healing, and results from the tissues inability to produce adequate amounts of pro-angiogenic factors. In these conditions, inadequate blood vessels grow, and circulation is not properly restored, increasing the risk of tissue death. The discovery of new pro- and anti-angiogenic factors as well as the development of ways to regulate endogenous pro- and anti-angiogenic factors provides a *novel* path for treatment of such conditions.

### Brain Angiogenesis Inhibitor Proteins and Fragments

The embodiments of the present disclosure encompass the BAI protein family, and in particular the protein BAI1, and its two cleavage products, the protein fragments Vstat120 and Vstat40. Embodiments and aspects of the present disclosure also describes the use of these polypeptides as anti-angiogenic and anti-tumorigenic therapy for gliomas, other types of cancer and conditions involving aberrant angiogenesis, such as, but not limited to, age-related macular degeneration.

The protein BAI1 and extracellular fragments thereof disclosed herein, including Brain Angiogenesis Inhibitor 1 (BAI1) (SEQ ID NO.: 1), and fragments Vstat120 (SEQ ID NOS.: 2 and 4) and Vstat40 (SEQ ID NOS.: 3 and 5) have anti-angiogenic properties. The disclosed proteins and fragments are part of the BAI family of proteins. The first of these proteins disclosed, BAI1, is a 170 kDa cell membrane protein and the gene encoding it is located on chromosome 8q24. BAI1 has a structure similar to that of a class B 7-transmembrane G-protein coupled receptor. Its putative ligand(s) are currently unknown. It has a 387 amino acid intracellular domain (about 45 kDa) and an unusually large extracellular domain (120 kDa) containing several definable domains or motifs, as schematically illustrated in Fig. 17A. These motifs include an Arg-Gly-Asp (RGD) integrin binding motif, five thrombospondin type 1 repeats (TSR), and a putative cleavage site found in G protein coupled receptors (GPS site). Less is known about the BAI1 homologues BAI2 and BAI3 although they are known to have a generally similar structure, have 7-transmembrane domains, also contain TSR regions (four only) but do not have an integrin RGD binding motif.

TSP1 contains three type I repeats. The type I repeat motif is more effective than the entire protein at inhibiting angiogenesis and contains two regions of activity. The amino-terminal end contains a tryptophan-rich motif that blocks fibroblast growth factor (FGF-2 or bFGF) driven angiogenesis. The second region of activity, the CD36 binding region of TSP1, can be found on the carboxy-terminal half of the type I repeats. Type I repeats have also been shown to bind to heparin, fibronectin, TGF-β, and others, potentially antagonizing the effects of these molecules on endothelial cells. Soluble type I repeats have been shown to decrease EC numbers by inhibiting proliferation and promoting apoptosis.

The extracellular domain of BAI1 can be cleaved at a G-protein coupled receptor proteolytic site (GPS) from BAI1, releasing the whole soluble 120 kDa fragment termed Vasculastatin-120 (Vstat120). The Vstat120 peptide contains both the RGD site in addition to all five TSRs and inhibits both *in vitro* and *in vivo* angiogenesis. The presence of an RGD binding domain allows for interactions with cell surface integrins involved in cell migration and intracellular growth factor signaling, while multiple TSR domains suggest anti-angiogenic properties based on the function of TSR in the thrombospondin-1 protein and the analysis of synthetic peptides with BAI1-derived sequence. Additionally, Vstat120 expression is strongly reduced during tumor growth in several aggressive malignant human glioma models.

A second cleavage site located between the first and second TSRs on BAI1's extracellular domain results in the protein fragment, Vasculostatin-40 (Vstat40). The size of the Vstat40 fragment is about 40 kDa based upon size markers in Western blotting experiments, as illustrated in Figs. 17A and 17B. The Vstat40 fragment retains the RGD motif distal to one of the TSRs. The presence of one TSR and an RGD motif in Vstat40 confers on Vstat40 anti-angiogenic properties.

The present disclosure relates that Vstat40 has inhibitory activity in *in vitro* assays for angiogenesis, cell proliferation and cell migration. Some evidence suggests that the two cleavage events that yield Vstat40 and Vstat120 may be mutually exclusive: once one fragment is generated, the other fragment can no longer be generated. Additionally, the cleavage event leading to Vstat40 appears to occur more readily, such that Vstat40 is produced more abundantly than Vstat120.

The amino acid sequence of BAI1 (SEQ ID NO.: 1), including the leader sequence thereof, is presented in Fig. 12. The sequences of Vstat120 (SEQ ID NO.: 2) and Vstat40 (SEQ ID NO.: 3), including leader sequences thereof are shown in Figs. 13 and 14 respectively. The sequences of Vstat120 (SEQ ID NO.: 4) and Vstat40 (SEQ ID NO.: 5), not including leader sequences thereof are shown in Figs. 15 and 16 respectively.

### Methods of Production

Embodiments of the present disclosure provide methods of production of the anti-angiogenic protein BAI1, and the Vstat120 and Vstat40 peptides. In one method, for example, Vstat120 may be synthesized via solid-phase peptide synthesis (SPPS). In SPPS, small beads are treated with linkers on which peptide chains such as Vstat120 or Vstat40 can be built. Once the entire peptide is assembled it is cleaved from the bead, filtered, and then purified. Another method suitable for producing either Vstat120 or Vstat40 is via prokaryotic or eukaryotic expression of the protein fragment, followed by purification of the protein product. The gene fragment encoding Vstat120 or Vstat40 and an inducible promoter operably linked thereto may be cloned into an expression vector plasmid and then transformed into a bacterial or eukaryotic host cell for expression therein. The resulting protein or fragment produced cells may then be collected and purified by methods well-known by those of skill in the art. The expression vector can also be introduced in eukaryotic cells and expressed therein to produce the desired proteins secreted into the culture media, from which they may be isolated.

### Methods of Use

Aspects of the present disclosure encompass methods of interfering, inhibiting, or disrupting angiogenesis via the use of the BAI1 protein, its fragments, or their homologues. Such inhibition can be accomplished by administration of Vstat120/40 or via regulation of the BAI1 protein.

One method for the treatment or prevention of abnormal angiogenesis may be by administering to a host, for example a mammal, in need of such treatment a pharmaceutical composition comprising the anti-angiogenic compounds Vstat120, Vstat40, the homologues of these two compounds resulting from the proteins BAI2 or BAI3, or a combinations thereof.

The methods of the disclosure further encompass treating or preventing cancer or a tumor in a host in need of such treatment by administering to the host the anti-angiogenic compounds Vstat120, Vstat40, or the homologues of these two compounds resulting from the proteins BAI2 or BAI3 or a combination thereof. It is contemplated that the administration could be via a number of protein delivery methods including but not limited to direct iv injection or through time-release capsules or nanoparticles.

Another contemplated method of the disclosure is for treating or preventing abnormal angiogenesis by modulating the expression of endogenous Vstat120, Vstat40, or their homologues in a cell by regulating the cleavage of these fragments from their parent proteins. Vstat40 is cleaved from BAI1 by a furin protease while the enzyme cleaving BAI1 at the GPS site to generate Vstat120 is currently unknown. Augmenting the release of these protein fragments from BAI1 by these proteases via administration of a compound or composition presents an avenue for delivery.

Yet another useful method provides a way of delivering the anti-angiogenic compounds Vstat120, Vstat40, or the homologues of these two compounds resulting from BAI2 or BAI3 via gene therapy or virotherapy. The genetic sequence encoding Vstat120 or Vstat40 or their homologues may be inserted into the genome using a viral vector to replace an "abnormal," disease-causing gene or to increase or restore expression of the protein fragments. Possible viruses which could be used as vectors include, but are not limited to, retroviruses, adenoviruses, or herpes simplex viruses. DNA encoding these compounds could also be delivered non-virally through direct introduction of therapeutic DNA into target cells, or by an artificial liposome or nanoparticle carrying the genetic sequence. The vector may be used to deliver genetic sequence to target cells including but not limited to glia in a patient. The gene is then incorporated into the target cells genome and a functional protein product, in this case the encoded Vstat peptide, is generated by the cell.

### Vasculostatin inhibits intracranial glioma growth and negatively regulates in vivo angiogenesis through a CD36-dependent mechanism

The cleaved and secreted 120 kDa Vstat120 fragment of BAI1, functions as an autonomous paracrine anti-angiogenic factor (Kaur et al., Oncogene, 2005;24: 3632-3642,). However, Vstat120 expression can also prolong the life of rats bearing intracranial gliomas. This tumor suppressive effect of Vstat120 in the brain was sustained even when glioma cells were engineered to over-express EGFRvIII, an oncogenic mutant EGFR resulting in highly angiogenic invasive and aggressive tumors (Nishikawa et al., Proc. Natl. Acad. Sci. U.S.A. 1994;91: 7727-7731,). These results highlight the potential significance of harnessing Vstat120 as a therapeutic agent for the treatment of the most malignant form of glioma in humans.

The mechanism of Vstat120 angiostatic effect is poorly understood (Kaur et al., Oncogene, 2005; 24: 3632-3642, Koh et al., Exp. Cell Res., 2004; 294: 172-184). The extracellular domain of BAI1 includes five TSRs, and an integrin binding RGD motif (Nishimori et al., Oncogene, 1997; 15: 2145-2150). TSRs were originally discovered in TSP-1, a naturally occurring potent inhibitor of angiogenesis. TSRs are approximately 60 amino acids in length and more than 180 different TSRs have been identified in over 70 TSR-containing proteins within the human genome (de Fraipont et al., Trends Mol Med, 2001; 7: 401-407). The latter include proteins of diverse functions such as the ADAMTS family of metalloproteases, complement factors C6, C7, C8, and C9, the F-, R- and M-Spondins, Semaphorins, Unc5, Heparin binding growth-associated molecule (HB-GAM), and BAI-1, -2 and -3. The levels of sequence identity between TSR within a single protein is as diverse as that found in other TSR-containing proteins, suggesting a complex evolutionary origin (Nicholson et al., Evol. Biol., 2005; 5: 11). The high level of heterogeneity in sequence between TSRs within and across diverse proteins suggests that they may carry out multiple functions. Based on current knowledge, homology in function cannot be inferred, but rather needs to be tested for each individual TSR. This highlights the importance of defining the function of individual TSRs in different proteins so that structural determinants can be identified in the future that will help accelerate the design of structure-function prediction algorithms. While at least five TSR-containing proteins: TSP-1 and -2, ADAMTS-1 and -8, and BAI1/Vstat120 are potent inhibitors of angiogenesis, so far only the TSR of TSPs have been convincingly linked to the anti-angiogenic activity of that protein family. Recent structural data have suggested that TSR-containing proteins could be sub-divided into two categories based on the number and orientation of the disulfide bonds between their three anti-parallel strands and the overall positive charge of their outer shell surface (Tan et al., J. Biol. Chem., 2007).

The first category encompasses TSP-1 and -2; BAI 1-3, and the ADAMTS proteins, while the second category comprises the F- and M-spondins, and some complement proteins. The fact that all known anti-angiogenic TSR-containing proteins belong to the first category, suggest that similarity in protein anti-angiogenic action might directly derive from homology in function of some of their TSR. Furthermore, the likely mechanistic basis of this distinction is the capacity to bind the anti-angiogenic endothelial cell receptor CD36. The studies disclosed herein support this hypothesis.

Purified recombinant peptides expressing three of the five TSRs of BAI1 were initially shown to inhibit angiogenesis in a rabbit corneal angiogenesis assay (Nishimori et al., Oncogene, 1997;15: 2145-2150). It remained unclear, however, whether they would serve the same function in the full length human BAI1 protein, where native conformation, post-translational modifications and physiological concentrations might define activity.

Data also showed that the TSRs of BAI1 are responsible for the recognition and engulfment of apoptotic cells by macrophages though the ELMO/Dock180/Rac signaling axis (Park et al., Nature, 2007). Further complicating the issue is a recent study suggesting that the angiostatic effect of BAI1 was mediated by its ability to block αvβ5 integrin receptors on endothelial cells (Nishimori et al., Oncogene, 1997; 15: 2145-2150; Koh et al., Exp Cell Res, 2004; 294: 172-184). Since the anti-angiogenic effects of thrombospondin-1 and -2 TSRs are mediated by their binding to CD36 and subsequent activation of a signaling cascade that triggers apoptosis (Dawson et al., J. Cell Biol., 1997; 138: 707-717, Jimenez et al., Nat. Med., 2000; 6: 41-48., Anderson et al., Cancer Biol. Ther., 2007; 6: 454-462), the anti-angiogenic effect of Vstat120 might equally be dependent on engagement of endothelial cell CD36 by Vstat120 TSRs. Vstat120 inhibits bFGF-induced migration of CD36-expressing HDMECs but not that of HUVECs, which do not express CD36. The ability of Vstat120 to inhibit HDMEC migration was suppressed in the presence of function-blocking CD36 antibodies.

Vstat120 also inhibited corneal neovascularization in wild type but not CD36 knockout mice. Combined, these results indicate that the inhibitory effect of Vstat120 is dependent on CD36 expression on endothelial cells both *in vitro* and *in vivo.* The anti-angiogenic effect mediated by thrombospondin-1 binding to CD36 is followed by sequential activation of p59*fyn*, caspase-3 like proteases and p38 mitogen activated protein kinases, and leads to endothelial cell apoptosis. The precise signaling cascade activated by Vstat120 interaction with CD36 in endothelial cells remains to be determined, but we predict that it will likely overlap with that elicited by TSP-1 given their homology in function.

The CLESH domain of CD36 is a critical determinant of the binding of TSP-1 and -2 TSR to endothelial cells. This domain may form part of a negatively charged loop within CD36 and may interact with the positively charged front groove of TSRs of type 1. The ability of Vstat120 to bind to purified peptides containing the CLESH domain of CD36, indicates a specific interaction between Vstat120 and CD36, and functional homology with TSP-1 and -2 TSR. These studies provide the first direct evidence that non-TSP TSRs of type 1 can bind CLESH domains. These results demonstrate the significance of the endothelial receptor, CD36 in mediating Vstat120's angiostatic effect. Thus, Vstat120 is dependent on the presence of CD36 to suppress the process of neovascularization both *in vitro* and *in vivo.*

### Vasculostatin-40 (Vstat40)

The present disclosure further encompasses the primary cleavage product of the BAI1 extracellular domain, which is an approximately 40 kDa in size secreted molecule, Vasculostatin-40 (Vstat40). Vstat40 contains one thrombospondin type 1 repeat (TSR), a domain capable of triggering anti-angiogenic responses by binding the CD36 receptor on endothelial cells. It also contains an RGD integrin-binding domain. The disclosure also provides that the protease furin mediates the Vstat40 processing and that Vstat40 inhibits migration and cord formation of CD36+ endothelial cells.

As shown in Fig. 12, the amino acid sequence of BAI1 (SEQ ID NO.: 1) comprises two cleavage sites, the first between the residues R328 and S329, and the second between L926 and S927. The first cleavage site releases Vstat40, which has the amino acid sequence SEQ ID NO.: 2 (Fig. 13) less the first approximately 32 leader sequence amino acids. Cleavage at the second cleavage site only releases the Vstat120 fragment which may include, as in SEQ ID NO.: 3 (Fig. 14), the leader sequence. Determination of the precise cleavage site of Vstat40 was performed using three approaches: 1) a broad region was defined by the approximate location of the cleavage based on Vstat40 40kDa size, 2) this region was subjected to a deletion scanning approach to refine the location of the cleavage (Fig. 18), 3) based on the refined cleavage location and the finding of a consensus cleavage site for the furin protease in this region point mutations were generated to identify the amino acids necessary for the cleavage. Truncation at amino acid 328 generates a product of the approximate size of Vstat40, as shown in Fig. 18A, indicating that the cleavage site is close to amino acid 328. Dashed vertical lines (Fig. 18A) indicate the site of Vstat40 cleavage. Fragment 1-374 was still cleaved and generated a low amount of Vstat40,

The attachment of 3 kDa tags (Fig. 18B, dark shade) to constructs ending between amino acids 322 and 334 constructs 2,3,4 still allowed cleavage into Vstat40, indicating that the cleavage site occurs between amino acids 322 and 330. The amino acid sequence S³²²-T³³⁰ was identified as a putative cleavage site for furin protease using a published algorithm (Duckert et al, Protein Engineering, Design and Selection vol 17, pp.107-112, 2004). To confirm that this sequence was required for the binding of a protease and to determine which amino acids were necessary for the cleavage, an alanine mutational scanning was perfomed in this region (Fig. 21). These experiments demonstrated that Q325A and R328A abolished the cleavage consistent with the importance of hydrophilic amino acids for furin processing, while S326A and S329A did not affect Vstat40 processing. The predicted cleavage site is between amino acids R328 and S329, consistent with the findings of the truncation mapping studies of Fig. 18.

To confirm the involvement of furin in the cleavage of Vstat40 a number of experiments were performed: 1) the use of furin inhibitors was shown to abrogate the cleavage into Vstat40 (Fig. 19), while matrix metalloproteinase inhibitors had no effect (Fig. 20), 2) the processing of BAI1 was monitored in furin-deficient LoVo cells with or without furin reconstitution (Fig.22). LoVo cells showed a strong reduction in the generation of Vstat40 which was restored upon furin cDNA transfection, suggesting that furin is the main enzyme responsible for cleavage.

The biological effects of Vstat40 and Vstat120 on the migration of endothelial cells, and especially of CD36+ endothelial cells are shown in Figs. 23-25C. The data of the present disclosure indicate that the anti-migratory effects of both of the secreted BAI1 Vstat fragments, Vstat40 and Vstat120, are mediated by the CD36 receptor on endothelial cells. An anti-αCD36-specific antibody abrogated the effects of Vstat40 and Vstat120 on endothelial cells, as shown in Figs. 24A and 24B.

One aspect of the present disclosure, therefore, is a polypeptide, wherein the amino acid sequence of the polypeptide has an amino acid sequence selected from the group consisting of SEQ ID NOS.: 3 and 4, or a conservative variant thereof, and wherein the polypeptide comprises an integrin binding domain and a thrombospondin type 1 repeat.

In one embodiment of this aspect of the disclosure, the polypeptide may have the amino acid sequence according to SEQ ID NO.: 3.

In another embodiment of this aspect of the disclosure, the polypeptide may have the amino acid sequence according to SEQ ID NO.: 4.

In one aspect, the polypeptide is isolated from an animal or a human.

In yet another aspectof the disclosure, the polypeptide may be isolated from a cell culture, wherein the cell culture may be comprised of animal or human cells comprising a heterologous nucleic acid encoding the polypeptide, and wherein the heterologous nucleic acid may be an expression vector comprising a region encoding the polypeptide operably linked to a gene expression regulatory region.

In one aspect, the cell culture may be comprised of animal or human cells.

In various aspects of the disclosure, the expression vector is selected from the group consisting of: a plasmid vector, a viral vector, and an artificial chromosome, and wherein the expression vector optionally is incorporated into the genomic DNA of the animal or human cells.

In another aspect, the cell culture is comprised of bacterial cells.

Another aspect of the present disclosure is an expression vector selected from the group consisting of: a plasmid vector, a viral vector, and an artificial chromosome, and wherein the expression vector comprises a heterologous nucleic acid encoding a polypeptide having an amino acid sequence selected from the group consisting of SEQ ID NOS.: 3 and 4, and conservative variants thereof, and wherein the polypeptide comprises an integrin binding domain and a thrombospondin type 1 repeat.

Another embodiment of the invention is an expression vector comprising a heterologous nucleic acid encoding a polypeptide that has the amino acid sequence of SEQ ID NO.: 3, SEQ ID NO.: 5, or conservative variants thereof having at least 80% identity to SEQ ID NO.: 3 or SEQ ID NO.: 5, wherein the polypeptide consists of an integrin binding domain and one thrombospondin type 1 repeat, and whereby the polypeptide binds CD36 via the thrombospondin type 1 repeat and inhibits angiogenesis.

In embodiments of this aspect of the disclosure, the polypeptide encoded by the heterologous nucleic acid has the amino acid sequence according to SEQ ID NO.: 3.

In other embodiments of the disclosure, the polypeptide encoded by the heterologous nucleic acid has the amino acid sequence according to SEQ ID NO.: 4.

Another embodiment of the disclosure provides methods of preparing a polypeptide, comprising: providing a first polypeptide, wherein the first polypeptide is BAI1 having an amino acid sequence according to SEQ ID NO.: 1, or an extracellular fragment thereof, wherein the extracellular fragment has a sequence selected from the group consisting of: SEQ ID NO.: 2, SEQ ID NO.: 4 and conservative variants thereof having at least 80% identity to SEQ ID NO.: 2 or SEQ ID NO;: 4; and contacting the first polypeptide with furin thereby forming a second polypeptide consisting of an integrin binding domain and at least one thrombospondin type 1 repeat.

In aspects of the method of this aspect of the disclosure, the first polypeptide may be according to SEQ ID NO.: 1, and the second polypeptide has an amino acid sequence selected from the group consisting of: SEQ ID NOS.: 2, 3, 4, 5, and conservative variants thereof.

In one embodiment of the disclosure, the first polypeptide may have the amino acid sequence according to SEQ ID NO.: 2, or conservative variants thereof having at least 80% identity to SEQ ID NO.: 2, and the second polypeptide may have the amino acid sequence according to SEQ ID NO.: 3 or conservative variants thereof having at least 80% identity to SEQ ID NO.: 3.

In another embodiment, the first polypeptide may have the amino acid sequence according to SEQ ID NO.: 4, or conservative variants thereof having at least 80% identity to SEQ ID NO.: 4, and the second polypeptide may have the amino acid sequence according to SEQ ID NO.: 5, or conservative variants thereof having at least 80% identity to SEQ ID NO.: 5.

In the aspect of the method of this aspect of the disclosure, the method may further comprise isolating the second polypeptide.

Yet another aspect of the disclosure are methods of inhibiting the proliferation of endothelial cells comprising: contacting a population of endothelial cells with a polypeptide having an amino acid sequence derived from that of the protein BAI1 (SEQ ID NO.: 1), wherein the amino acid sequence of the polypeptide has an amino acid sequence selected from the group consisting of: SEQ ID NOS.: 3 and 5, or conservative variants thereof, and wherein the cleavage product comprises an integrin binding domain and a thrombospondin type 1 repeat, whereby contacting the endothelial cells with the polypeptide inhibits the proliferation of the endothelial cells.

In this aspect of the disclosure, the population of endothelial cells may be in an animal or human, and the method may further comprise systemically administering the polypeptide to the animal or the human.

In one aspect of the disclosure, the method may further comprise directly delivering the polypeptide to a population of cells in the animal or the human.

Another aspect of the disclosure provides methods of inhibiting angiogenesis comprising: contacting a population of endothelial cells with a polypeptide, wherein the polypeptide has an amino acid sequence selected from the group consisting of: SEQ ID NOS.: 2, 3, 4, 5, or conservative variants thereof, and wherein the polypeptide comprises an integrin binding domain and at least one thrombospondin type 1 repeat, whereby contacting the endothelial cells with the polypeptide inhibits the proliferation of the endothelial cells thereby inhibiting angiogenesis.

In one embodiment of the disclosure, the polypeptide may bind to the CD36 receptor on the surface of endothelial cells.

In this aspect of the disclosure, the method may further comprise delivering the polypeptide to an animal or human, whereby angiogenesis is inhibited in the animal or human.

In various aspects of the method of this aspect of the disclosure, the polypeptide may be delivered to an animal or human as a bolus or as a sustained delivery.

In one aspect of this method, the polypeptide may be delivered to an animal or human by administering thereto a pharmaceutically acceptable composition comprising a nucleic acid vector incorporating therein a heterologous nucleic acid sequence encoding a polypeptide having an amino acid sequence selected from the group consisting of: SEQ ID NOS.: 2, 3, 4, 5, or conservative variants thereof; and expressing the heterologous nucleic acid sequence, thereby delivering the polypeptide to the endothelial cells.

In various embodiments of this method of the disclosure, the nucleic acid vector may be a plasmid vector or a viral vector.

In aspects of the method, the angiogenesis in the animal or human is a result of a pathological condition.

In aspects of this method of the disclosure, the pathological condition may be a tumor, a wound, or age-related macular degeneration.

Still another aspect of the disclosure provides methods of inhibiting the formation of a tumor in an animal or human, wherein the tumor is sustained or disseminated by angiogenesis, comprising: contacting a developing tumor in an animal or human with a polypeptide derived from the protein BAI1 (SEQ ID NO.: 1), wherein the amino acid sequence of the polypeptide may have an amino acid sequence selected from the group consisting of: SEQ ID NOS.: 2, 3, 4, 5, and wherein the polypeptide comprises an integrin binding domain and at least one thrombospondin type 1 repeat, whereby contacting the tumor with the polypeptide inhibits angiogenesis by binding to the CD36 receptor on endothelial cells, thereby inhibiting the formation of the tumor.

In this aspect of the disclosure, in embodiments of the method, the tumor may be a tumor of the brain.

In one aspect, the tumor is a glioma.

In this aspect of the disclosure, the method may further comprise directly delivering the polypeptide to the tumor of the brain by injection into the tumor tissue or injection into a blood vessel leading into the tumor.

Another embodiment of the disclosure is a pharmaceutical composition comprising a polypeptide comprising an integrin binding domain and one thrombospondin type 1 repeat, wherein the amino acid sequence of the polypeptide is selected from the group of SEQ ID NO.: 3, SEQ ID NO.: 5, and conservative variants thereof having at least 80% identity to SEQ ID NO.: 3 or SEQ ID NO.: 5, whereby the polypeptide binds CD36 via the thrombospondin type 1 repeat and inhibits angiogenesis.

Preferably, the polypeptide has the amino acid sequence of SEQ ID NO.: 3.

Still another aspect of the disclosure provides a pharmaceutical composition comprising an isolated polypeptide derived from the protein BAI1 (SEQ ID NO.: 1), wherein the amino acid sequence of the polypeptide may have at least 80% similarity with a sequence selected from the group consisting of: SEQ ID NOS.: 2, 3, 4, 5, or conservative variants thereof, and comprises an integrin binding domain and at least one thrombospondin type 1 repeat, and a pharmaceutically acceptable carrier.

It should be emphasized that the embodiments of the present disclosure, particularly, any "preferred" embodiments, are merely possible examples of the implementations, merely set forth for a clear understanding of the principles of the disclosure. Many variations and modifications may be made to the above-described embodiment(s) of the disclosure without departing substantially from the spirit and principles of the disclosure. All such modifications and variations are intended to be included herein within the scope of this disclosure, and the present disclosure and protected by the following claims.

The following examples are put forth so as to provide those of ordinary skill in the art with a complete disclosure and description of how to perform the methods and use the compositions and compounds disclosed and claimed herein. Efforts have been made to ensure accuracy with respect to numbers (*e.g*., amounts, temperature, *etc.*), but some errors and deviations should be accounted for. Unless indicated otherwise, parts are parts by weight, temperature is in °C, and pressure is at or near atmospheric. Standard temperature and pressure are defined as 20 °C and 1 atmosphere.

### EXAMPLES

The specific examples below are to be construed as merely illustrative, and not limitative of the remainder of the disclosure in any way whatsoever. Without further elaboration, it is believed that one skilled in the art can, based on the description herein, utilize the present disclosure to its fullest extent.

### Example 1

### Expression of Vstat120 suppresses the growth of intracranial gliomas

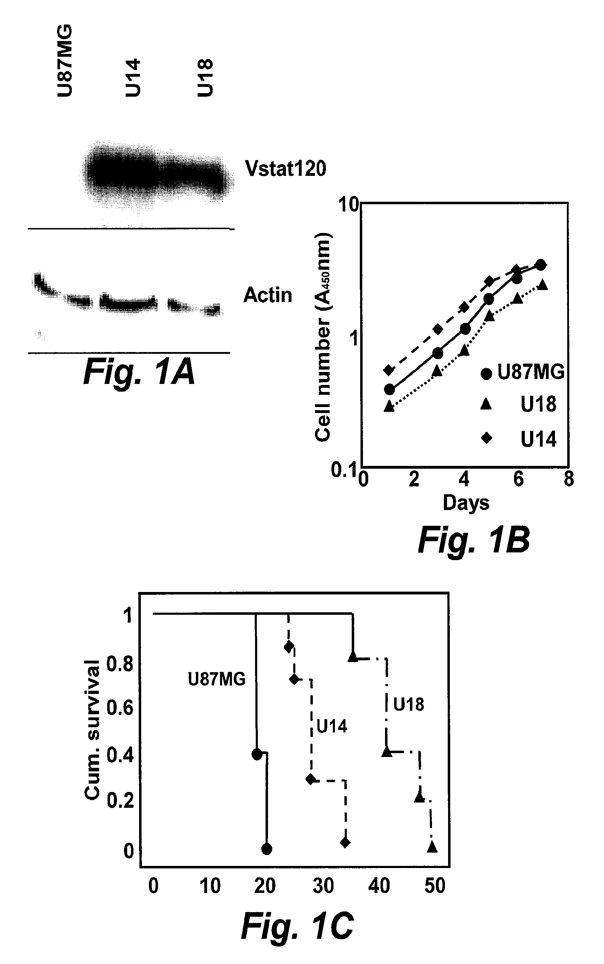

Since BAI1 is predominantly expressed in the brain, the role of its proteolytic cleavage product, Vstat120, on the orthotopic growth of brain tumors was examined. Two clones (U14 and U18) stably expressing Vstat120 in human U87MG glioma cells following transfection with an expression vector were generated, as shown in Fig. 1A. The *in vitro* proliferation rates of these cells were not altered by Vstat120 expression (see Fig. 1 B).

To assess the therapeutic potential of Vstat120 expression on tumor growth in the brain, U14, U18 and parental U87MG cells (106/animal) were stereotactically implanted into the brains of athymic nude rats (n=6 rats/group). Both Vstat120 expressing clones extended the median survival of animals compared to the control parental U87MG cells (p<0.05). The median survival of animals injected with control parental U87MG glioma cells was 18 days. In contrast, the median survival of the animals injected with Vstat120 expressing clones U14 and U18 was 28 (24-34) & 41 (34-49) days, days, respectively as shown in Fig. 1C.

### Example 2

### Expression of Vstat120 can suppress intracranial tumor growth even when a proangiogenic stimulus is present

Parental U87MG cells are pro-angiogenic due to the loss of PTEN (Wen et al., Proc. Natl. Acad. Sci. U.S.A, 2001; 98: 4622-4627). However, they lack expression of EGFRvIII, the genetic hallmark of a large subset of GBM (Ekstrand et al., Proc. Natl. Acad. Sci. U.S.A, 1992; 89: 4309-4313; Libermann et al., Nature, 1985; 313: 144-147). This mutation confers an aggressive and highly angiogenic phenotype (Nishikawa et al., Proc. Natl. Acad. Sci. U.S.A, 1994; 91: 7727-7731; Abe et al., Cancer Res., 2003; 63: 2300-2305). To test if Vstat120 could suppress the growth of such highly aggressive gliomas, we utilized U87MG glioma cells that stably express the EGFRvIII mutant receptor (U87ΔEGFR)(Nishikawa et al., Proc. Natl. Acad. Sci. U.S.A, 1994; 91: 7727-7731). U87ΔEGFR was then stably transfected with a Vstat120 expression vector and selected two clones (Δ19 and Δ22) for further analyses. These cells expressed Vstat120, and did not show any alterations in their *in vitro* proliferation rate (Fig. 2A). To assess the therapeutic potential of Vstat120 expression on this highly aggressive glioma, both the mouse subcutaneous and rat orthotopic xenograft models were used.

Athymic *nu*/*nu* mice (n=6/group) injected with U87ΔEGFR cells formed large subcutaneous tumors, and mice had to be sacrificed by day 25. In contrast, mice injected with Vstat120 expressing cells Vstat120 were unable to form detectable tumors under the skin (Fig. 2B). To examine whether Vstat120 would also antagonize tumor formation in their orthotopic microenvironment we implanted 106 cells of U87MG, U87ΔEGFR, Δ19, and Δ22 cells stereotactically in the brain of athymic *nu*/*nu* rats. Initially, the effect of Vstat120 on tumor growth, was measured non-invasively by magnetic resonance imaging (MRI) on day 14 to determine tumor growth (n=3/group). Immediately thereafter the animals were sacrificed and corresponding sections of the brains were analyzed by hematoxylin and eosin (H&E) staining. The results indicated smaller tumors in gliomas derived from cells expressing Vstat120 (Fig. 2C). Next, we examined the effect of Vstat120 expression on survival of animals with intracranial tumors (n=6 animals/group). Expression of EGFRvIII to U87MG cells significantly reduced the median survival of rats from 31 to 21 days (p < 0.002). In contrast, the median survival of rats implanted with intracranial Δ19 and Δ22 cells was 57 (34 to 114 days) and 41 (days 37 to 45) days, respectively (Fig. 2D).

These results demonstrated that expression of Vstat120 significantly slowed tumor growth and increased survival (p <0.001 between animals implanted with either of the Vstat120 expressing clones (Δ19, and Δ22) and the control U87ΔEGFR cells). Interestingly, among rats injected with Vstat120 expressing Δ19 cells there were three long term survivors who lived for more than 60 days after tumor cell implantation. Two of these three rats eventually died of tumor burden on days 85 and 114, and the third animal was sacrificed on day 168 and found to be tumor free. Combined, the above results demonstrate that while Vstat120 has potent inhibitory effects on glioma growth *in vivo,* both in the subcutaneous and brain microenvironments.

### Example 3

### Measurement of vascular density in intracranial gliomas

To determine whether the reduced ability of Vstat120 expressing cells to grow *in vivo* but not *in vitro* was due to impairment in recruiting the vascular supply needed for solid tumor growth, we examined the vascular phenotype of intracranial tumors derived from U87ΔEGFR and Vstat120 clones. Immunohistochemistry for von Willebrand Factor (vWF), a vascular marker, revealed a significant reduction in the density of vascular structures in Vstat120-expressing tumors (Fig. 3A). Vessel density in brain tumor sections derived from Vstat120 expressing cells (Δ19 and Δ22) showed an average of 18 (±2.0) vessels/mm2 while control U87ΔEGFR tumors had 32 (±1.5) vessels/mm2 (p<0.005) (Fig. 3B). These data demonstrate that Vstat120 can reduce the vascular density of a very aggressive form of human brain tumor in its orthotopic microenvironment in a rat model.

### Example 4

### Vstat120-mediated inhibition of endothelial cell migration in vitro requires CD36

The above data indicate that Vstat120 can suppress angiogenesis by antagonizing the neovascularization response of endothelial cells. CD36 is known to be expressed on human dermal microvascular endothelial cells (HDMECs) but not on human umbilical vein endothelial cells (HUVECs). To evaluate the role played by CD36 on Vstat120 anti-angiogenic effect, we compared the susceptibility of CD36 expressing HDMECs and non expressing HUVECs to the inhibitory effects of Vstat120 in a transwell migration assay. Treatment of endothelial cells with conditioned media (CM) from 293 cells expressing Vstat120 (Fig. 4A) inhibited the migration of CD36 expressing HDMECs, but had no effect on CD36 non expressing HUVEC cell migration, potentially implicating CD36 in Vstat120 effects (Fig. 4B). The effect of Vstat120 on HDMEC migration was further tested in a scratch-wound migration assay (Figure 4C). Confluent HDMECs were wounded and the extent of wound closure was measured after 8 hr, at which time the cells were fixed and stained. The leading edge of cells migrated a greater distance in the control cells compared to the Vstat120-treated cells. Quantification of the distance migrated, percent wound closure and migrations speed of cells showed that Vstat120 significantly reduced the migration of HDMECs.

Next, we determined whether an anti-CD36 function-blocking antibody could abrogate the inhibitory function of Vstat120 on HDMECs in a scratch-wound migration assay (Fig. 4D). Confluent HDMECs were left untreated or were treated with anti- CD36 antibody for 30 minutes prior to treatment with control or Vstat120 containing CM. Quantification of these results showed that pre-incubation of HDMECs with neutralizing anti-CD36 antibody completely abrogated the anti-migratory effect of Vstat120.

### Example 5

### Vstat120 can bind to the CLESH domain of CD36.

The anti-angiogenic effects of TSP-1 and -2 mediated by their binding to CD36 on endothelial cells is dependent upon the binding of the TSR domain(s) to a conserved region within CD36 called the CLESH domain. The ability of Vstat120 to bind to recombinant CD36 CLESH domain peptides was tested. GST-tagged peptides spanning amino acids 5 to 143, and 67 to 157 of CD36 were expressed and purified in *E.coli* (Figs. 5A and 5B). The indicated GST fusion proteins were tested for their ability to bind to TSP1. Briefly the indicated recombinant peptide bound to glutathione-sepharose beads was used to pull down TSP1 from CM of LN229 cells constitutively expressing TSP1 (clone C9). Western blot of the pulled down proteins confirmed their ability to bind to TSRs (Fig. 5C, left panel). The indicated GST fusion proteins were then tested for their ability to bind to Vstat120 in a similar pull down assay using Vstat120 from CM of LN229 glioma cells constitutively expressing Vstat120. Fig. 5C shows that the GST-tagged recombinant CD36 CLESH proteins, but not GST alone, pulled down Vstat120. These results demonstrate the ability of Vstat120 to bind to CLESH domain of CD36.

### Example 6

### Vstat120 inhibits corneal angiogenesis in a CD36-dependent fashion

To examine whether Vstat120 would also inhibit neovascularization *in vivo,* corneal angiogenesis assays in mice were performed. To further assess the involvement of CD36 in this process, the effects of Vstat120 on bFGF induced corneal angiogenesis in wild type and CD36 knockout mice were compared. Micropellets containing human bFGF and CM of 293 cells transfected with Vstat120 cDNA or a control vector were implanted into the mice cornea. The results show that Vstat120 can reduce the extent of bFGF-induced corneal neovascularization in wild type mice (Fig. 6A). This inhibitory effect was completely abolished in CD36 knockout mice. The mean area of neovascularization in corneas with pellets containing Vstat120 CM was significantly decreased (40%, p<0.05) as compared to those containing control CM (Fig. 6B). Altogether, these results show that CD36 expression is required for Vstat120 anti-angiogenic effects on endothelial cells both *in vitro* and *in vivo.*

### Example 7

### Culture of cell lines and transfection conditions:

The human glioblastoma (U87MG, LN229) and 293 cell lines were previously described (Ishii et al., Brain Pathol, 1999;9: 469-479). U87ΔEGFR stably express the EGFRvIII mutant form of EGFR. The LN229Vstat120 (clone 13) and LN229TSP1 (clone C9) cells were prepared by stably transfecting LN229 cells with expression vectors for Vstat120 (pcDNA3.1mychisVstat120) and TSP1 (pcDNATS1). Conditioned media (CM) from cells was prepared from 80% confluent cultures grown for 48-96 hours in serum free media. For transient transfections 293 cells plated on 60 mm2 dishes were transfected with 8ug of lacZ/pcDNA3.1, Vstat120-myc/his pcDNA3.1, or wild-type BAI1pcDNA3.1 vector, using GenePORTER (Gene Therapy Systems; Cat. # T201007) transfection reagent. 4 mL serum-free CM was collected from cells after 48 hours and stored at - 20°C. The CM (4ml) was precipitated using 50% TCA and resuspended in 150 µL 1x Laemmli sample buffer.

### Example 8

### Preparation of recombinant GST fusion proteins

Two different GST/CD36 constructs (Figure 5A) containing the CLESH domain (spanning amino acids 5-143, and 67-157) were prepared as previously described (34). All constructs were verified by direct nucleotide sequencing. The GST fusion proteins were expressed in *E. coli* BI21(DE3) bacteria. At log phase IPTG was added to a final concentration of 3 mM. Protein expression was carried out for 3 hrs at 37°C. The cells were then centrifuged at 16,000 g for 5 min, resuspended in 5ml of lysis buffer (PBS + one Complete mini EDTA-free protease inhibitor cocktail tablet (#4693159, Roche) + 1 mg/ml lysozyme) and frozen overnight at -20°C. The cell solutions were thawed in warm water and pulse sonicated for 3 bursts of 15 seconds each. Cell lysates were sedimented at 31,000 g for 30 min, and the protein-rich pellets were washed successively with wash buffer 1 (25ml of PBS, 0.1% Triton X-100), wash buffer 2 (50 mM NaH2PO4, 300 mM NaCl, pH 8.0), and dissolved in 5ml of denaturing buffer (8 M urea, 50 mM Tris-HCl, pH 8.0). After 30 min centrifugation at 30,000g to remove insoluble debris, the proteins were refolded by drop wise addition into 20ml of refolding buffer (50mM Tris-HCl, 1 mM DTT, 1 mM EDTA, pH 9.0), then dialyzed overnight in 50mM tris pH 8.0. Recombinant proteins were bound to glutathione sepharose 4B resin (GE Healthcare) and were used for Vstat120/TSP1 pull down assays or purified by elution with 50 mM Tris-HCl 10 mM glutathione, pH 8.0.

### Example 9

### Glutathione-S-transferase pull-down assay

The GST-CD36-CLESH fusion protein solutions (15ml) were pre-absorbed with 100 µl of glutathione sepharose 4B beads (GE Healthcare) for 2 h at 4°C. After two washes with cold PBS, 15ml of undiluted CM (collected after 96h in serum-free medium) from stably transfected or parental control cells was added to 100 µl of beads and then incubated at 4°C overnight with constant rotation. The beads were centrifuged (100 g for 1 min) and washed twice with 5 ml of PBS. Bound proteins were eluted and solubilized in 50 µl of SDS-PAGE denaturing sample buffer.

### Example 10

### Western blot analysis

Immunoblots were performed on cell lysates (lysed in 8M Urea, 4% SDS, in 10mM Tris (pH 7.4), from indicated cells or tissue. Equal amounts of protein (40 µg) were resolved on a 7.5% SDS PAGE followed by transfer to nitrocellulose membranes. Western blots were probed with an anti-N-terminal BAI1 antibody, followed by goat anti-rabbit secondary antibody (DAKO Co. Carpinteria CA; Cat # P0448). Actin blots were probed with goat anti-actin antibodies (cat# SC-1616 SANTA CRUZ Biotechnology Inc. Santa Cruz, CA; diluted 1:500) followed by swine anti-goat secondary antibodies (cat# 605275 ROCHE Molecular Biochemicals, Indianapolis, IN; diluted 1:1000), and visualized by enhanced chemiluminescence (PIERCE Rockford IL). Anti-GST monoclonal antibody (Chemicon International, MAB3317) was used to detect the fusion proteins by Western blot.

### Example 11

### Proliferation assays

Proliferation rates of the different Vstat120-transfected and empty vector clones were assessed by a crystal violet assay. Equal numbers of cells (4,000) from each clone were plated in a 96 well plate (n=8). The cells were fixed with 1% glutaraldehyde, and then stained with 0.5% crystal violet. After washing, the crystals were dissolved in Sorenson's buffer (0.025M sodium citrate, 0.025M citric acid in 50% ethanol) and absorbance was read at A590 nm.

### Example 12

### Tumorigenicity studies

Subcutaneous tumor xenografts were performed as previously described. For intracranial studies, we stereotactically injected 106 glioma cells into the brains of athymic nude rats (average body weight of 150g) to establish orthotopic brain tumor xenografts as described. These animals were deeply anesthetized by administering an intraperitoneal injection of Ketamine (80mg/kg)/Xylazine (10mg/kg) mixture. The anesthetized animal was secured to a stereotactic frame and body temperature maintained by a heating pad. A saggital midline incision was made from 5 mm anterior to the bregma to the occiput. A 2 mm drill was then used to make a burr hole 3 mm to the right and 1 mm anterior of the bregma of the skull. A 23 gauge Hamilton syringe was advanced to a depth of 4.5 mm over a period of one minute, and then retracted 0.5 mm in order to form a pocket for injection and 5µl of tumor cell suspension was injected over a period of two minutes. After the injection, the needle was retracted over a period of one minute, and the burr hole was filled with sterile bone wax. The surface of the skull was washed with sterile water to destroy by osmosis any cells leaked into the subgaleal space. The scalp was subsequently closed with 3-0 running vicryl stitches. The animal was kept isolated during the recovery period. All animal studies were done in accordance with the Kaplan-Meier survival curves were compared using the log-rank test. A *P* value less than 0.05 was considered statistically significant. All statistical analyses were performed with the use of SPSS statistical software (version 14.0; SPSS Inc, Chicago, IL). The rodents were marked by a simple tattooing procedure for identification. All animal studies were done in accordance with guidelines issued by Emory University Institutional Animal Care and Use Committee.

### Example 13

### Magnetic resonance imaging

MRI scans were carried out on a 3T MRI scanner (Philips Intera) using a small volume coil (5-cm dia.). Animals were anesthetized as above and then placed in the coil. The head was secured using foam padding to minimize possible motions. MRI contrast agent, Gadolinium diethylenetriamine-pentaacetic acid (Gd-DTPA), was administrated iv. at a dose of 0.2 mM/kg to obtain signal enhancement in the tumor. Multi-slice T1-weighted spin echo images were obtained in the coronal orientation using a repetition time of 400 ms, echo time of 14 ms and imaging matrix of 128x128 with the field of view of 50 x 50 mm2. To match the histological analysis, a slice thickness of 2 mm was used without slice gap. Number of signal average was 3 for most of the scans. T1 weighted spin echo imaging was done before and after administration of the contrast agent for each animal using the same imaging parameters.

### Example 14

### Histological analysis and Immunohistochemistry

The harvested tumors were fixed in 10% buffered formalin followed by paraffin embedding and routine sectioning (8 µm). The sections were stained for von Willebrand Factor with a rabbit polyclonal antibody (1:4 dilution, Dako, Carpenteria, CA) to visualize the endothelial cells lining the blood vessels perfusing the tumor. The number of vascular structures/mm2 in the tumor xenografts was quantified by counting three different 10x microscopic fields for each of 3 rats/group. The three fields were averaged in each tumor and the averages for each animal used to give the final count +/- SEM.

### Example 15

### In vitro Transwell and scratch-wound endothelial cell migration assays

CM from HEK 293 cells transfected with Vstat120 (pcDNAecBAI1myc-his) or control vector (pcDNA3.1-LacZ, a β-galactosidase expression vector) was collected and concentrated 40x using a YM-10 filter (Amicon). For the Transwell migration assays, indicated cells were plated in Transwell modified Boyden chambers (Becton Dickinson Labware # 353097) with a pore size of 8 um (50,000 cells/chamber). The cells were placed on 1% serum-containing media overnight and were then pretreated with CM (diluted at 1x in endothelial medium) for 30 min. Media containing 10% serum was used as a chemoattractant in the bottom chamber, while CM remained in the upper chamber. After 8hrs migrated cells were quantified by counting three 10x microscopic views/filter and the data presented as means of 3 filters. For the scratch-wound migration assays, confluent HDMECs cultures were incubated in 1% media overnight in 12 well plates, then wounded with a 10µl pipette tip. Detached cells were removed by PBS washes and then treated with an anti-CD36 function blocking antibody at 10 µg/mL for 30 min (FA6- 152, Cell Sciences). The cells were treated with CM at a final concentration of 1x for 30 min, followed by incubation in 10% serum to induce cell migration. Initial wound width was measured, and the cells were allowed to migrate for 8h, and were then fixed with 1% glutaraldehyde, stained with 0.5% crystal violet, and photographed. The experiment was repeated 2 independent times and significance determined by Student's T test.

### Example 16

### In vivo cornea angiogenesis assays

Pellets were generated by mixing sterile solutions of bFGF (Research Diagnostics, Inc) at a final concentration of 25 ng/pellet, concentrated CM (50ng total protein from CM per pellet) and sucralfate (Teva Pharmaceuticals, North Wales, PA), and then spreading the solution onto nylon mesh-3-300/50 with an approximate pore size 0.4 x 0.4 mm (Tetko, Lancaster, NY). The mixture was sealed on both sides with hydron. In this case, 7.5 µl concentrated media /100 pellets were added. Individual pellets were detached by peeling the nylon mesh, and pellets of similar size were chosen under a dissecting microscope for implantation. Mice were anesthetized as above and the eyes were topically anesthetized with 0.5% proparacaine and 2% alocril and the globes proptosed with a forceps. Pellets were implanted approximately 1 mm from the limbus. Briefly, under a dissecting microscope, a central, intrastromal linear keratotomy (approximately 0.5 mm in length) was performed with a surgical blade (Bard-Parker #15; Becton Dickinson), and using the arm of a fine forceps, a micro-pocket was created toward the limbus. Pellets were placed at the base of the pocket. Erythromycin antibiotic ointment was applied to the operated eye, both to prevent infection and to decrease irritation. Mice received Buprenex (2.5 mg/kg subcutaneously) after surgery to control for pain. Five days post implantation, mice were anesthetized and 50ul of a 2.5mg/ml solution of sterile FITC-dextran (~ MW 70,000Da, Sigma) was injected into the retro-orbital sinus. The eyes were proptosed as before, and digital images of the cornea were captured under a fluorescent dissecting microscope (Leica) and transferred to Adobe Photoshop for measurements. The maximum vessel length and the neovascularization zone (in clock hours), were used to calculate the area of neovascularization, using the formula: Area (mm2) = 0.2 x π x VL (mm) x CH. VL = vessel length; CH = clock hours, where one clock hour = 30° of an arc.

### Example 17

To determine whether BAI1 expression is disrupted during tumorigenesis, the expression of the BAI1 protein in normal human brain cells and in Glioblastoma Multiforme (GBMs) was examined. An anti-BAI1 antibody was generated and using this antibody, immunohistochemical staining was done on human autopsy specimens containing GBMs. BAI1 was widely expressed in the normal brain tissue, but absent in the majority of the 18 human GBMs tissues investigated, indicating that the expression of BAI1 protein is likely lost or suppressed during tumor formation, as shown in Fig. 7A.

Additionally we compared BAI1 mRNA expression in 28 glioma cell lines versus expression in normal brain tissue using reverse transcription-polymerase chain reaction (RT/PCR). BAI1 mRNA was only expressed in normal brain tissue, and not found in any of the glioma cell lines, shown in Fig. 7B.

Neural stem cells along with normal astrocytes are the two cell types believed to be the origin of GBMs. RT/PCR was also used to measure BAI1 mRNA expression in mouse C17.2 cells, an immortal neural stem cell line, and compared to a line of Human Embryonic Kidney (HEK) 293 cells transfected with human BAI1 (hBAI1) cDNA. Western blotting was then used to compare expression of the hBAI1 and mouse BAI1 (mBAI1) proteins. Both mBAI1 mRNA and mBAI1 were found to be expressed by the C17.2 cells indicating BAI1 is expressed normally in neural stem cells and that disruption of its expression may be crucial to its role in tumorigenesis (see Fig. 7B).

### Example 18

The extracellular domain of BAI1 is proteolytically cleaved at a G-protein-coupled receptor proteolytic cleavage site, releasing the soluble fragment Vstat120. This fragment contains both an arginine-glycine-aspartic acid (RGD) integrin binding motif which is distal to five thrombospondin type 1 repeat (TSR) domains. To show that Vstat120 has anti-angiogenic properties, Vstat120 was tested using both *in vitro* (via a Boyden chamber assay and a crystal violet assay) and *in vivo* (via a subcutaneous matrigel plug assay) measurements of angiogenesis.

In the Boyden chamber assay, cultured human dermal microvascular endothelial cells (HDMECs) were incubated with conditioned media (CM) from LN229 GBM cells transiently transfected with an expression vector encoding the 120 kDa BAI1 fragment or an empty vector. The cells were then treated with basic fibroblast growth factor (bFGF) and cell migration was assayed using a modified Boyden chamber. The results show that condition media containing the 120 kDa fragment significantly reduced endothelial cell migration, as shown in Fig. 8A. Next, to examine its effect on EC proliferation, HDMECs were incubated with conditioned media from the same cells as above. The cells were then treated with bFGF and proliferation of the cells was determined using the crystal violet assay. Conditioned media, containing the 120kDa fragment (*), inhibited in vitro EC proliferation as shown in Fig. 8B.

To further examine whether expression of the 120kDa fragment would also affect *in vivo* angiogenesis, the mouse matrigel plug assay was used. Vector control or the 120kDa fragment-expressing LN229 cells were injected with matrigel subcutaneously in *nu*/*nu* mice. At 14 days after injection, the plug was removed and analyzed for vascular development by histology. We observed a robust vascular channel formation in the control plugs, while it was strikingly reduced in those containing the 120kDa fragment-expressing cells. These structures were vascular channels as they were lined with EC that stained with von Willebrandt factor (vWF) and perivascular smooth muscle and pericytes that stained with smooth muscle actin (SMA). The average length of the vascular channels/mm2 was determined, showing that plugs expressing the 120kDa fragment had only 11% of the average vascular channel length observed in the control plugs. As a control, the expression of the 120kDa fragment in both cell types was verified. Collectively, these data suggest an anti-angiogenic role for the 120kDa fragment of BAI1; and we named it Vasculostatin-120 (Vstat120). (See Fig. 9).

### Example 19

### Evidence for the antiangiogenic properties of Vstat40

To examine whether the Vstat40 cleavage fragment of BAI1 possessed anti-angiogenic functions like those of Vstat120, the following three *in vitro* assays were conducted: the endothelial cord formation assay, the Transwell migration assay (equivalent to the modified Boyden chamber assay) and the scratch wound assay. Vstat40, like Vstat120, contains an RGD integrin binding motif as well as two thrombospondin type 1 repeat (TSR) domains.

In the endothelial cord formation assay, HDMECs were grown on matrigel containing CM from cells transfected with LacZ (control), Vstat40 or Vstat120 cDNA. After 8 hrs, the number of cords and enclosed structures were counted. CM from Vstat40 transfected cells resulted in fewer enclosed structures signifying decreased angiogenesis. The Transwell migration chamber assay involved plating human umbilical vein endothelial cells (HUVEC) and HDMECs in Transwell migration chambers, placing them on 1% serum media overnight and then pretreating with CM from cells transfected either with LacZ, Vstat40, or Vstat120 cDNA for 30 min. Media containing 10% serum was used as a chemoattractant and then placed on the bottom of the chamber. After 8 h the migrated cells were quantified. No change in the number of migrating cells was determined using HUVECs, but a significant decrease in migrating cells was found for HDMECs.

A scratch wound assay was conducted to examine if an anti-CD36 function antibody, known to prevent anti-angiogenic functioning in thrombospondin-1 (which contains 3 type I TSR repeats) would prevent wound anti-angiogenic function in Vstat40 treated cells. Cultures of HDMECs were placed on 1% media overnight and then wounded using a 10 µl pipette tip. The cells were then left untreated or treated with anti-CD36 function blocking antibody at 10 µg/mL for 30 min. The cells were next treated with CM from cells transfected either with LacZ, Vstat40, or Vstat120 cDNA for 30 min, followed by treatment with 10% serum to induce cell migration. Final wound width and distance migrated was then measured after 8 h. Cells treated with CM from cells expressing Vstat40 migrated significantly less than those treated with CM from LacZ expressing cells. When retreated with an anti-CD36 function antibody, the inhibition of migration exhibited by these cells was blocked. Together, the results of the three assays indicate that Vstat40 possess anti-angiogenic properties, as shown in Fig. 10.

### Example 20

Treatment of endothelial cells with Vascular Endothelial Growth Factor (VEGF), also called vascular permeability factor (VPF), disrupts the adherence junctions between cells. The ability to inhibit such disruption, currently reduced by administering dexamethasone, would therefore be a useful addition to the anti-angiogenic properties of a compound. Cultures of HDMEC cells were treated with CM from cells transfected either with LacZ, Vstat40, or Vstat120 cDNA for 30 min, followed by treatment with VEGF or non treatment. Cells were then fixed and immunostained with an anti-VE (vascular endothelial) cadherin antibody revealed by a FITC antibody. The presence of VE-cadherin in the cell membrane was then measured by immunofluorescence. Pretreatment with the CM of Vstat40 transfected cells resulted in increased VE-cadherin-mediated cell-cell adherence compared to controls, indicating a preservation of adherence junctions between HDMEC cells, as shown in Fig. 11.

It should be emphasized that the above-described embodiments of the present disclosure are merely possible examples of implementations, and are merely set forth for a clear understanding of the principles of this disclosure. Many variations and modifications may be made to the above-described embodiment(s) of the disclosure without departing substantially from principles of the disclosure. All such modifications and variations are intended to be included herein within the scope of this disclosure and protected by the following claims.

## Claims

1. A pharmaceutical composition comprising a pharmaceutically acceptable carrier and a polypeptide comprising an integrin binding domain and one thrombospondin type 1 repeat, wherein the amino acid sequence of the polypeptide is selected from the group of SEQ ID NO.: 3, SEQ ID NO.: 5, and conservative variants thereof having at least 80% identity to SEQ ID NO.: 3 or SEQ ID NO.: 5, whereby the polypeptide binds CD36 via the thrombospondin type 1 repeat and inhibits angiogenesis.

2. The pharmaceutical composition of claim 1, wherein the polypeptide has the amino acid sequence of SEQ ID NO.: 3.

3. An expression vector comprising a heterologous nucleic acid operably linked to a gene regulatory region, said heterologous nucleic acid encoding a polypeptide that has the amino acid sequence of SEQ ID NO.: 3, SEQ ID NO.: 5, or conservative variants thereof having at least 80% identity to SEQ ID NO.: 3 or SEQ ID NO.: 5, wherein the polypeptide consists of an integrin binding domain and one thrombospondin type 1 repeat, and whereby the polypeptide binds CD36 via the thrombospondin type 1 repeat and inhibits angiogenesis.

4. A method of preparing a polypeptide inhibiting angiogenesis, comprising:
providing a first polypeptide, wherein the first polypeptide is BAI1 having an amino acid sequence according to SEQ ID NO.: 1, or an extracellular fragment thereof, wherein the extracellular fragment has a sequence selected from the group consisting of: SEQ ID NO.: 2, SEQ ID NO.: 4, and conservative variants thereof having at least 80% identity to SEQ ID NO.: 2 or SEQ ID NO.: 4; and contacting the first polypeptide with furin thereby forming a second polypeptide consisting of an integrin binding domain and one thrombospondin type 1 repeat.

5. The method of claim 4, wherein first polypeptide has the amino acid sequence according to SEQ ID NO.: 2, or conservative variants thereof having at least 80% identity to SEQ ID NO.: 2, and the second polypeptide has the amino acid sequence according to SEQ ID NO.: 3 and conservative variants thereof having at least 80% identity to SEQ ID NO.: 3, or the first polypeptide has the amino acid sequence according to SEQ ID NO.: 4, or conservative variants thereof having at least 80% identity to SEQ ID NO.: 4, and the second polypeptide has the amino acid sequence according to SEQ ID NO.: 5, and conservative variants thereof having at least 80% identity to SEQ ID NO.: 5

## Patentansprüche

1. Pharmazeutische Zusammensetzung umfassend einen pharmazeutisch annehmbaren Träger und ein Polypeptid, das eine Integrin-bindende Domäne und ein Thrombospondin Typ 1-Repeat enthält, wobei die Aminosäuresequenz des Polypeptids ausgewählt ist aus der Gruppe aus SEQ ID NO.: 3, SEQ ID NO.: 5 und konservative Varianten davon mit mindestens 80% Identität zur SEQ ID NO.: 3 oder SEQ ID NO.: 5, wobei das Polypeptid über das Thrombospondin Typ 1-Repeat CD36 bindet und Angiogenese inhibiert.

2. Pharmazeutische Zusammensetzung nach Anspruch 1, wobei das Polypeptid die Aminosäuresequenz der SEQ ID NO.: 3 aufweist.

3. Expressionsvektor umfassend eine heterologe Nukleinsäure, die operativ an eine genregulatorische Region gebunden ist, wobei besagte heterologe Nukleinsäure für ein Polypeptid kodiert, welches die Aminosäuresequenz SEQ ID NO.: 3, SEQ ID NO.: 5 aufweist, oder konservative Varianten davon mit mindestens 80% Identität zur SEQ ID NO.: 3 oder SEQ ID NO.: 5, wobei das Polypeptid aus einer Integrin-bindenden Domäne und einem Thrombospondin Typ 1-Repeat besteht, und wobei das Polypeptid über das Thrombospondin Typ 1- Repeat CD36 bindet und Angiogenese inhibiert.

4. Verfahren zur Herstellung eines Polypeptids, das Angiogenese inhibiert, umfassend:
Bereitstellen eines ersten Polypeptids, wobei das erste Polypeptid BAI1 mit einer Aminosäuresequenz entsprechend SEQ ID NO.: 1 ist oder ein extrazelluläres Fragment davon, wobei das extrazelluläre Fragment eine Sequenz besitzt ausgewählt aus der Gruppe bestehend aus: SEQ ID NO.: 2, SEQ ID NO.: 4, und konservative Varianten davon mit mindestens 80% Identität zur SEQ ID NO.: 2 oder SEQ ID NO.: 4, und Kontaktieren des ersten Polypeptids mit Furin, wodurch ein zweites Polypeptid gebildet wird, das aus einer Integrin-bindenden Domäne und einem Thrombospondin Typ 1-Repeat besteht.

5. Verfahren nach Anspruch 4, wobei das erste Polypeptid eine Aminosäuresequenz entsprechend SEQ ID NO.: 2 aufweist oder konservative Varianten davon mit mindestens 80% Identität zur SEQ ID NO.: 2 und das zweite Polypeptid eine Aminosäuresequenz entsprechend SEQ ID NO.: 3 und konservativer Varianten davon mit mindestens 80% Identität zur SEQ ID NO.: 3 aufweist oder das erste Polypeptid eine Aminosäuresequenz entsprechend SEQ ID NO.: 4 aufweist oder konservative Varianten davon mit mindestens 80% Identität zur SEQ ID NO.: 4, und das zweite Polypeptid eine Aminosäuresequenz entsprechend SEQ ID NO.: 5 aufweist und konservative Varianten davon mit mindestens 80% Identität zur SEQ ID NO.: 5.

## Revendications

1. Composition pharmaceutique comprenant un vecteur pharmaceutiquement acceptable et un polypeptide comprenant un domaine de liaison à l'intégrine et une répétition de thrombospondine de type 1, dans lequel la séquence d'acides aminés du polypeptide est choisie dans le groupe comprenant SEQ ID NO : 3, SEQ ID NO : 5 et leurs variants conservatifs présentant une identité d'au moins 80 % avec SEQ ID NO : 3 ou SEQ ID NO : 5, moyennant quoi le polypeptide se lie à CD36 par l'intermédiaire de la répétition de thrombospondine de type 1 et inhibe l'angiogenèse.

2. Composition pharmaceutique selon la revendication 1, dans laquelle le polypeptide possède la séquence d'acides aminés de SEQ ID NO : 3.

3. Vecteur d'expression comprenant un acide nucléique hétérologue lié de manière fonctionnelle à une région de régulation des gènes, ledit acide nucléique hétérologue codant un polypeptide qui possède la séquence d'acides aminés de SEQ ID NO : 3, de SEQ ID NO : 5 ou de leurs variants conservatifs présentant une identité d'au moins 80 % avec SEQ ID NO : 3 ou SEQ ID NO : 5, dans lequel le polypeptide est constitué d'un domaine de liaison à l'intégrine et d'une répétition de thrombospondine de type 1, et moyennant quoi le polypeptide se lie à CD36 par l'intermédiaire de la répétition de thrombospondine de type 1 et inhibe l'angiogenèse.

4. Procédé de préparation d'un polypeptide inhibant l'angiogenèse, comprenant :
la fourniture d'un premier polypeptide, dans lequel le premier polypeptide est BAI1 possédant une séquence d'acides aminés selon SEQ ID NO : 1, ou un fragment extracellulaire de celui-ci, dans lequel le fragment extracellulaire possède une séquence choisie dans le groupe constitué par : SEQ ID NO : 2, SEQ ID NO : 4 et leurs variants conservatifs présentant une identité d'au moins 80 % avec SEQ ID NO : 2 ou SEQ ID NO : 4 ; et le contact du premier polypeptide avec une furine pour ainsi former un second polypeptide constitué d'un domaine de liaison à l'intégrine et d'une répétition de thrombospondine de type 1.

5. Procédé selon la revendication 4, dans lequel le premier polypeptide possède la séquence d'acides aminés selon SEQ ID NO : 2, ou des variants conservatifs de celle-ci présentant une identité d'au moins 80 % avec SEQ ID NO : 2, et le second polypeptide possède la séquence d'acides aminés selon SEQ ID NO : 3 et des variants conservatifs de celle-ci présentant une identité d'au moins 80 % avec SEQ ID NO : 3, ou le premier polypeptide possède la séquence d'acides aminés selon SEQ ID NO : 4, ou des variants conservatifs de celle-ci présentant une identité d'au moins 80 % avec SEQ ID NO : 4, et le second polypeptide possède la séquence d'acides aminés selon SEQ ID NO : 5 et des variants conservatifs de celle-ci présentant une identité d'au moins 80 % avec SEQ ID NO : 5.
